# EUROPÄISCHE PATENTSCHRIFT

(11) **EP 3 634 946 B1**
(45) Veröffentlichungstag und Bekanntmachung des Hinweises auf die Patenterteilung: **14.09.2022**
(21) Anmeldenummer: 18727840.3
(22) Anmeldetag: 06.06.2018
(51) Int. Cl.: C07C 263/10, C07C 265/14

(54) **VERFAHREN ZUR HERSTELLUNG VON ISOCYANATEN**
PROCESS FOR THE PRODUCTION OF ISOCYANATES
PROCÉDÉ DE FABRICATION D'ISOCYANATES

(30) Priorität: 08.06.2017 EP 17175068
(43) Veröffentlichungstag der Anmeldung: 15.04.2020
(73) Patentinhaber: Covestro Intellectual Property GmbH & Co. KG, 51373 Leverkusen (DE)
(72) Erfinder: TAUBE, Wolfgang, 41470 Neuss (DE); BRUNS, Rainer, 51467 Bergisch Gladbach (DE); MAUSBACH, Artur, 41541 Dormagen (DE)
(74) Vertreter: Levpat
(86) Internationale Anmeldenummer: PCT/EP2018/064851
(87) Internationale Veröffentlichungsnummer: WO 2018/224530

(56) Entgegenhaltungen:
- EP-A1- 1 935 875
- WO-A1-2013/029918

## Beschreibung

Die Erfindung betrifft ein Verfahren zur Herstellung eines Isocyanats durch Phosgenierung des korrespondierenden primären Amins in der Gasphase, bei welchem das gasförmige Reaktionsproduktgemisch durch Inkontaktbringen mit mindestens einem Strom einer Quenchflüssigkeit in einer Quenchzone abgekühlt wird, wobei die insgesamt eingesetzte Quenchflüssigkeit, bezogen auf ihre Gesamtmasse, organische Lösungsmittel in einem Anteil von maximal 50,0 Massen-% umfasst, wobei der Rest zu 100Massen-% aus mindestens dem herzustellenden Isocyanat besteht, unter Erhalt eines Gemisches aus Reaktionsproduktgemisch und Quenchflüssigkeit; wobei ferner das so erhaltene Gemisch aus Reaktionsproduktgemisch und Quenchflüssigkeit in einer Sammelzone in eine flüssige und eine gasförmige Phase getrennt wird, wobei ein flüssiger Lösungsmittelstrom umfassend mehr als 50,0 Massen-% an organischen Lösungsmitteln, bezogen auf die Gesamtmasse des flüssigen Lösungsmittelstroms, in die Sammelzone und/oder in einen strömungstechnisch nach der Sammelzone angeordneten Sammeltank für die in der Sammelzone erhaltene flüssige Phase eingeleitet wird.

Die Herstellung von Isocyanaten, insbesondere Diisocyanaten, in der Gasphase ist schon seit längerem im Stand der Technik beschrieben und wird industriell insbesondere zur Herstellung von Toluylendiisocyanat, Xylylendiisocyanat, 1,5-Pentandiisocyanat, 1,6-Hexamethylendiisocyanat, Isophorondiisocyanat und Diisocyanatodicyclohexylmethan genutzt. In allen Verfahren fällt ein gasförmiges Rohprodukt an, das mindestens Isocyanat, Chlorwasserstoff und gegebenenfalls nicht umgesetztes Phosgen (Phosgen wird im Allgemeinen im Überschuss eingesetzt) umfasst, und das weiter aufgearbeitet werden muss, um das gewünschte Isocyanat in Reinform zu gewinnen.

Ein solches Verfahren ist beispielsweise in EP 0 289 840 B1 beschrieben. Die in einem Rohrreaktor gebildeten Diisocyanate sind bei den Reaktionstemperaturen von bis zu 500 °C thermisch nicht stabil. Eine rasche Abkühlung der Reaktionsgase nach der Phosgenierungsreaktion auf Temperaturen unter 150 °C ist daher notwendig, um die Bildung unerwünschter Nebenprodukte durch den thermischen Zerfall von Diisocyanat oder durch eine Weiterreaktion zu vermeiden. In EP 0 289 840 B1 oder EP 0749 958 B1 wird dazu das den Reaktionsraum kontinuierlich verlassende gasförmige Gemisch, welches u. a. Diisocyanat, Phosgen und Chlorwasserstoff enthält, in ein inertes Lösungsmittel, z. B. Dichlorbenzol, eingeleitet.

Weiterhin sind Verfahren bekannt, die zur Abkühlung der Reaktionsgase Wärmeaustauscher einsetzen und/oder die Gase im Vakuum entspannen (DE 101 58 160 A1).

Im Stand der Technik ist es heute bevorzugt, die Abkühlung der Reaktionsgase durch Eindüsen von unter den Reaktionsbedingungen inerten Flüssigkeiten unter möglichst vollständiger Kondensation des gebildeten Isocyanats zu bewirken. Dies geschieht in einer sog. *Abkühl- oder Quenchzone.* Ziel ist es, während der Abkühlung eine Weiterreaktion in der Flüssigphase zu unerwünschten Nebenprodukten (wie zum Beispiel Isocyanurate, Biurete, Allophanate, Carbodiimide oder Harnstoffe) weitgehend zu unterbinden.

Im Verfahren gemäß WO 2005/123665 A1 wird versucht, die Verweilzeit zwischen Reaktionsende und Abkühlzone dadurch zu verringern, dass sich zwischen der Reaktionszone und der Zone, in der der Reaktionsabbruch herbeigeführt wird, ein Bereich mit reduziertem Strömungsquerschnitt befindet. Als einzudüsende Flüssigkeiten eignen sich Lösungsmittel, Isocyanat oder Gemische aus Lösungsmittel und Isocanat.

Auch in WO 2011/003532 A1 wird ein Verfahren zum raschen Abkühlen des gasförmigen Reaktionsgemisches mittels Eindüsen einer Quenchflüssigkeit in das kontinuierlich aus der Reaktionszone in die nachgeschaltete Quenchzone strömende Gasgemisch offenbart. Als geeignete Quenchflüssigkeiten werden Lösungsmittel, Isocyanat oder Lösungsmittel-Isocyanat-Gemische genannt.

Das Eindüsen von Quenchflüssigkeit mit Hilfe von mindestens zwei Sprühdüsen, welche am Eingang der Quenchzone angeordnet sind, wird in EP 1 403 248 A1 offenbart. Hierbei eignen sich als Quenchflüssigkeiten organische Lösungsmittel oder eine Mischung verschiedener organischer Lösungsmittel, welche mit dem gebildeten Diisocyanat nicht reagieren. Eine Lösung des gebildeten Diisocyanats in einem geeigneten organischen Lösungsmittel kann auch verwendet werden, was die Menge von eingesetztem Lösungsmittel reduziert. In diesem Fall beträgt der Volumenanteil des Lösungsmittels an der Quenchflüssigkeit von 40 bis 90 %, d. h. der Volumenanteil des Isocyanats beträgt bis zu 60 %. Der Durchmesser der Quenchzone kann größer oder kleiner sein als der Durchmesser der Reaktionszone.

Optimiert wird dieses System gemäß der Lehre von EP 1 935 875 A1 dadurch, dass zum Abbruch der Reaktion das Reaktionsgemisch aus dem Reaktionsraum heraus durch eine Kühlstrecke geführt wird, in die hinein in zwei Zonen Flüssigkeiten eingedüst werden, sodass die direkte Kühlung in der Kühlstrecke einstufig (d. h. in der Terminologie dieser Anmeldung: nur ein einziges Kondensationsgemisch erhaltend) in zwei oder mehreren hintereinander geschalteten Kühlzonen erfolgt. Das erzeugte Diisocyanat wird also in einem gemeinsamen Kondensationsgemisch erhalten. Dieses Gemisch wird vorzugsweise in einem Flüssigkeitssammelbehälter, angeordnet unterhalb der Kühlstrecke, aufgefangen. Dieses Kondensationsgemisch kann zur Abtrennung des hergestellten Isocyanats ausgeschleust oder, bevorzugt nach erfolgter Kühlung, teilweise auf eine oder mehrere Kühlzonen der Kühlstrecke zurückgeführt werden. Der auf diese Weise zurückgeführte Strom, der der Kühlflüssigkeit der zweiten Kühlzone entspricht, enthält insbesondere 30 bis 90 Gew.-% Lösungsmittel und 10 bis 90 Gew.-Isocyanat. Die Zugabe eines Lösungsmittelstroms in den Flüssigkeitssammelbehälter oder einen diesem nachgelagerten Sammeltank für das Kondensationsgemisch wird in dieser Schrift nicht offenbart.

Neben dem Kondensationsgemisch im Sammelbehälter wird nach der Kühlstrecke ein Gasstrom, enthaltend wenigstens Chlorwasserstoff, Phosgen, gegebenenfalls Lösungsmittel, und das hergestellte Isocyanat, erhalten. Dieser Gasstrom wird dem Sammelbehälter entnommen und einer Waschkolonne zugeführt und dort weitgehend von seinen Isocyanatanteilen befreit. Bevorzugt erfolgt diese Wäsche im Gegenstrom mit Lösungsmittel. Die so erhaltene Waschphase, bestehend aus Diisocyanat und zum überwiegenden Teil aus Lösungsmittel (80 bis 99,99 Gew.-%), wird in einer bevorzugten Ausführungsform als Quenchflüssigkeit der *ersten* Kühlzone der Kühlstrecke eingesetzt. In der/den nachgeschalteten Kühlzone(n) wird, wie oben bereits geschildert, bevorzugt das Kondensationsgemisch aus dem Sammelbehälter (das 10 bis 70 Gew.-% Isocyanat und 30 bis 90 Gew.-% Lösungsmittel enthält) als Quenchflüssigkeit eingesetzt. Realistischerweise ist zu erwarten, dass bei dieser Verfahrensweise (Lösungsmittelanteil der in der ersten Kühlzone eingesetzten Kühlflüssigkeit nicht unter 80 Gew.-% und Lösungsmittelanteil der in der zweiten Kühlzone und ggf, weiteren Kühlzonen eingesetzten Kühlflüssigkeit jeweils nicht unter 30 Gew.-%) der Lösungsmittelanteil in der *insgesamt* eingesetzten Kühlflüssigkeit regelmäßig mehr als 50 Gew.-% beträgt.

In EP 1 935 876 A1 wird ebenfalls der Einsatz verschiedener geeigneter Quenchflüssigkeitsströme erwähnt. Dabei wird auch auf den Einsatz der Waschflüssigkeit aus der Gaswäsche der den Kondensatsammelbehälter nach der Quenche verlassenden Brüden als Quenchflüssigkeit hingewiesen. Ferner wird die Möglichkeit, Isocyanat als Quenchflüssigkeit einzusetzen, erwähnt (Absatz [0032]).

Auf mehrere Kühlzonen in der Quench-Stufe weist auch EP 2 196 455 A1 hin. Auf den integrierten Verbund der Kühlzonen mehrerer Reaktoren mit einer Quench-Stufe wird hier erstmals hingewiesen. Auch hier wird die Möglichkeit, Isocyanat als Quenchflüssigkeit einzusetzen, erwähnt (Absatz [0055]).

Die Anmeldung WO2007/014936 A2, Verfahren zur Herstellung von Isocyanaten (in der Gasphase), beschreibt eine Quenchzone, in welcher durch Eindüsen einer Quenchflüssigkeit das gasförmige Rohprodukt rasch abgekühlt wird. In dieser Quenchzone wird das Reaktionsgemisch, das im Wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570 °C auf 100 bis 200 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im Wesentlichen vollständig in der Gasphase verbleiben. Als mögliche Quenchflüssigkeiten werden Lösungsmittel, Isocyanat und Lösungsmittel-Isocyanat-Gemische genannt. Erwähnt wird das Eindüsen einer Quenchflüssigkeit zum Abkühlen des Reaktionsgemisches und selektiven Lösen des gebildeten Diisocyanats im Lösungsmittel, wobei eine erste Trennung in eine Flüssigphase und eine Gasphase mit überwiegend Phosgen und Chlorwasserstoff als Bestandteilen erfolgt. Die beiden Phasen werden danach einer entsprechenden Aufarbeitung zugeführt. Auf Optimierungsmöglichkeiten dieses Verfahrensschrittes wird nicht eingegangen.

WO 2010/063665 A1 verweist auf ein mögliches Problem der bisher genannten Quenche-Varianten. Wird zumindest ein Teil der Quenchflüssigkeit aus dem Sammelbehälter nach der Quenche, also das flüssige rohe Produktgemisch, entnommen, besteht die Möglichkeit, dass Feststoffe enthalten sein können, die die Quench-Düsen verstopfen können. Es werden verschiedene Techniken, wie z. B. Zentrifugieren, Abdestillieren des zur Quenche vorgesehenen Flüssigkeitsanteils oder Filtrieren beschrieben. Um die Temperatur des ausgewählten Quenche-Stromes für die gestellte Aufgabe einzustellen, kann der Strom mittels eines Wärmeaustauschers gekühlt, bzw. aufgeheizt werden. Diese Schrift offenbart (S. 12, Z. 6 bis 9, S. 13, zweiter Absatz) verschiedene Quellen für das Quenchmedium: Einen aus dem der Quenche 3 nachgeschalteten Phasentrenner 9 abgezweigten Teilstrom 15 (der notwendigerweise auch in der Quenche verflüssigtes Isocyanat enthält), frisches Lösungsmittel 19, einen Teil der im Phasentrenner gewonnenen Flüssigphase 13 sowie einen Teilstrom des zweiphasigen Produktstroms 7.

In WO 2010/115908 A2 wird eine spezielle Ausführungsform der Quenche offenbart. Um Folgereaktionen des Reaktionsgases bei bzw. nach der Quenchestufe zu verhindern, werden die Quenchedüsen und deren Anordnung derart konzipiert, dass eine weitgehend vollständige Benetzung der Wandung im Quenchebereich erfolgt. Damit wird das gesamte Reaktionsgemisch erfasst. Als Quenchflüssigkeiten werden Lösungsmittel sowie Gemische mit Isocyanat bzw. Rohgemisch der Phosgenierreaktion, gegebenenfalls nach Partikelentfernung, vorgeschlagen. Der Anteil an Isocyanat in der Flüssigkeit, mit der die Wandungen benetzt werden bzw. im Quenchmedium kann im Bereich von 0 bis 100 Prozent liegen (S. 11, Z. 5 bis 8).

EP 2 463 273 A1 offenbart eine Prozessvariante für Isocyanatkonzentrationen von größer als 70 Massen-% im die Quenchzone verlassenden flüssigen Sumpfprodukt. Der die Quenchzone gasförmig verlassende Strom wird ohne Durchlaufen einer Waschkolonne direkt in einen mantelgekühlten Kondensator geleitet. Der verbleibende Gasstrom wird direkt der Phosgenrückgewinnung zugeführt. Trotz der hohen Temperatur und hohen Isocyanatkonzentration im flüssigen Sumpfprodukt der Quenchzone werden über Isocyanat-Restgehalte im verbleibenden Gasstrom keine Aussagen gemacht. Der Kondensatstrom wird mit dem Kondensat des Dampfstroms, der durch Entspannung des flüssigen Sumpfproduktes aus der Quenchzone entsteht, vereinigt und als Quenchflüssigkeit zurückgeführt.

WO 2013/029918 A1 befasst sich mit einem Verfahren zur Herstellung von Isocyanaten durch Umsetzung der korrespondierenden Amine, enthalten in mindestens einem Eduktstrom A, mit Phosgen, enthalten in mindestens einem Eduktstrom P, in einer Reaktoranlage umfassend mindestens eine Mischzone und mindestens eine Reaktionszone, wobei Eduktstrom A und/oder Eduktstrom P gegebenenfalls einen oder mehrere inerte Stoffe enthalten, und wobei in Zeiträumen, in denen der Mengenstrom S^{x} der eingesetzten Amine unterhalb des Mengenstroms S⁰ der eingesetzten Amine bei Betrieb bei der Nennkapazitat der Reaktoranlage liegt, im Vergleich zum Betrieb bei der Nennkapazitat des Reaktors (i) das Verhältnis von Phosgen zu Amin erhöht wird und/oder (ii) die Konzentration des inerten Stoffs oder der inerten Stoffe in dem Amin enthaltenden Eduktstrom A und/oder dem Phosgen enthaltenden Eduktstrom P erhöht wird.

Diese Patentanmeldung beschreibt unter anderem auch die Durchführung der Phosgenierung in der Gasphase. Dabei wird, um die Bildung unerwünschter Nebenprodukte zu reduzieren beziehungsweise zu vermeiden und weiterhin auch eine Zersetzung des gebildeten Isocyanats zu unterbinden, das Reaktionsgas vorzugsweise unmittelbar nach der Reaktion in einer Quenchzone abgekühlt. Hierzu wird ein vorzugsweise flüssiges Quenchmedium zugegeben. Durch Verdampfung des Quenchmediums nimmt dieses Wärme auf, was zu einer schnellen Abkühlung des Reaktionsgases führt.

Das zur Kühlung eingesetzte Quenchmedium enthält vorzugsweise ein Lösungsmittel. In diesem Fall ist es bevorzugt, *dem Quenchmedium vor dessen Zugabe* in die Quenchstufe das Lösungsmittel zuzugeben, um Lösungsmittelverluste im Quenchmedium auszugleichen. Die Zugabe eines Lösungsmittelstroms in eine der Quenchzone nachgelagerte Sammelzone, in welcher die Abtrennung des verdampften Quenchmediums von der verbleibenden, das Isocyanat enthaltenden Flüssigphase erfolgt, wird in dieser Anmeldung nicht offenbart.

In einer anderen Ausführungsform gemäß WO 2013/029918 A1 ist es ebenfalls möglich, dass das Quenchmedium einen Teil des in der Quenchzone abgekühlten Produktstroms enthält oder aus diesem besteht.

WO 2014/122180 A1 befasst sich mit einer Verfahrensführung, welche den völligen Verzicht auf eine Rezyklierung des in der Quenchzone erhaltenen Kondensationsgemisches in die Quenche ermöglicht. Zu diesem Zweck wird die in der Quenche erhaltene Gasphase vor Einleitung in eine Waschkolonne partiell kondensiert, und das erhaltene Kondensat wird anstelle rezyklierten Kondensationsproduktes als zusätzliche Quenchflüssigkeit verwendet.

WO 2015/011070 A1 befasst sich mit der Vermeidung von Ablagerungen im Bereich der Quenche. Es wird gelehrt, die Temperatur der Wand der Reaktionszone unmittelbar oberhalb der Quenchzone auf einen Wert zu halten, der maximal 15 % unter der in Kelvin angegebenen Maximaltemperatur der Wand der Reaktionszone liegt. Als geeignete Quenchflüsskeit werden Lösungsmittel, Isocyanat und Gemische aus Lösungsmittel und Isocyanat genannt.

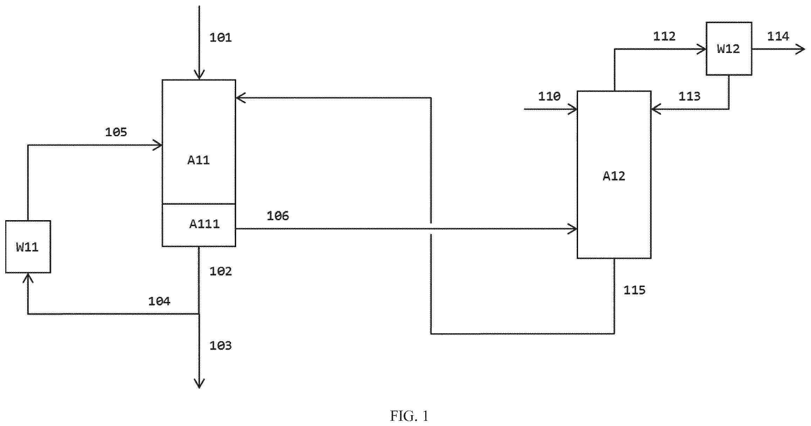

Zusammenfassend kann festgehalten werden, dass im Stand der Technik das Eindüsen von unter den Reaktionsbedingungen inerten Flüssigkeiten in den Rohproduktgasstrom die bevorzugte Variante zur raschen Abkühlung und Kondensation des Produkts darstellt. Dabei hat sich die mindestens zweistufige Quenche, in welcher in einer - in Strömungsrichtung des Produktgasgemisches betrachtet - ersten Quenchestufe eine überwiegend Lösungsmittel enthaltende Quenchflüssigkeit und in einer nachgeschalteten zweiten Quenchestufe eine zu beträchtlichen Anteilen aus dem zu bildenden Isocyanat bestehende Quenchflüssigkeit verwendet wird, als besonders vorteilhaft herausgestellt. Eine mögliche Ausführungsform dieser Verfahrensführung ist im Folgenden anhand der ersten Abbildung (FIG. 1) erläutert:
Das gasförmige Rohprodukt (101), vorwiegend bestehend aus Isocyanat, Chlorwasserstoff und überstöchiometrisch eingesetztem Phosgen, wird in der Quenche (A11) durch Eindüsen von Quenchflüssigkeit (105 und 116) schnell abgekühlt, um unerwünschte Folgereaktionen zu vermeiden. Der den Sammelbehälter (A111) im unteren Bereich der Quenche flüssig verlassende Strom (102), enthaltend vor allem Isocyanat und Quenchflüssigkeit (insbesondere 10 bis 70 Gew.-Isocyanat und 30 bis 90 Gew.-% Lösungsmittel, vgl. EP 1 935 875 A1, Absatz [0042]), wird zum Teil zur Produktreinigung geleitet und zum Teil über den Quenchekühler (W11) als Quencheflüssigkeit zur Quenche zurückgeführt.

Der die Quenche gasförmig verlassende Stoffstrom (106), enthaltend vor allem verdampfte Quenchflüssigkeit, Chlorwasserstoff und Phosgen, wird einer Waschkolonne (A12) zugeführt, um Restgehalte an Isocyanat möglichst weitgehend aus dem Brüdenstrom zu entfernen. Je größer der Gehalt an Isocyanaten im zugeführten Stoffstrom (106) ist, desto höher muss der am Kopf der Waschkolonne zugeführte Waschflüssigkeitsstrom (Lösungsmittel) gewählt werden, und desto mehr Trennstufen werden für eine zuverlässige Rückhaltung benötigt. Der Waschflüssigkeitsstrom setzt sich aus dem Kondensat (113) des Kondensators (W12) und zusätzlichem Lösungsmittel (110) zusammen. Der praktisch Isocyanat-freie Brüdenstrom (114) enthält vor allem Phosgen und Chlorwasserstoff. Der flüssige Sumpfablauf (115) enthält vor allem Lösungsmittel und wird zweckmäßigerweise als Quencheflüssigkeit der Quenche zugeführt.

Die betriebliche Erfahrung hat gezeigt, dass der Betrieb der Quenche auf diese Art und Weise, obwohl grundsätzlich möglich und vielen anderen Verfahrensführungen überlegen, nicht frei von Nachteilen ist. Insbesondere wurde, entgegen jedweder Erwartung, beobachtet, dass sich insbesondere *im Bereich der in Strömungsrichtung des Produktgasgemisches ersten, vorwiegend mit Lösungsmittel betriebenen Quenche* mit der Zeit Ablagerungen bilden, die im schlimmsten Fall eine Abschaltung und Reinigung des Reaktors erforderlich machen können. Ferner gelangen in der Reaktions- oder Quenchezone entstehende oder darin eingetragene Feststoffe sowie Schwersieder mit dem flüssigen Quencheprodukt in den Quenchekühler (W11) und die Quenchedüsen und können dort Verschmutzungen verursachen. Zudem wird bei der Eindüsung eines stark (mehr als 50%tiger Lösungsmittelanteil) Lösungsmittel-haltigen Stroms in die Quenchezone durch die Verdampfung eines Teils des Lösungsmittels ein großer Brüdenstrom erzeugt, der erheblich zum Gesamtvolumen des die Quenchestufe verlassenden Gasstromes beiträgt und auf diese Weise die Abtrennung von in diesem Gasstrom mitgerissenem Isocyanat aufwändiger macht, etwa indem dieser eine größere Dimensionierung der eingesetzten Apparate bedingt. Außerdem besteht die Gefahr, dass durch die große Brüdenmenge evtl. vorhandene feste Ablagerungen aus dem Reaktor in die nachgeschaltete Abtrennung von Isocyanat aus dem in der Quenche erhaltenen gasförmigen Strom mitgerissen werden und dort zu Verstopfungen in Apparaten führen.

Aufbauend auf diesem Stand der Technik bestand daher ein Bedarf an einer weiteren Optimierung der Quenche eines gasförmigen Isocyanat-Rohprodukts. Insbesondere war es erstrebenswert, die Quenche dahingehend zu verbessern, dass Ablagerungen im Quenchebereich vermieden oder zumindest vermindert werden, und zwar ohne die Qualität und Verarbeitbarkeit des erhaltenen Kondensationsproduktes signifikant zu beeinträchtigen. Ferner war es wünschenswert, den Gehalt an Lösungsmittel im in der Quenche erhaltenen Kondensationsprodukt möglichst gering zu halten, weil das dort enthaltende Lösungsmittel in der Aufarbeitung wieder abgetrennt werden muss.

Dem vorstehend Gesagten Rechnung tragend ist ein Gegenstand der vorliegenden Erfindung ein Verfahren zur Herstellung eines Isocyanats durch Phosgenierung des korrespondierenden primären Amins in der Gasphase umfassend die Schritte:
(i) Bereitstellung eines gasförmigen Stroms eines primären Amins;
(ii) Bereitstellung eines gasförmigen Phosgenstroms;
(iii) Vermischen des gasförmigen Stroms des primären Amins aus Schritt (i) und des gasförmigen Phosgenstroms aus Schritt (ii) zu einem gasförmigen Reaktionsgemisch unter Einhaltung eines auf primäre Aminogruppen bezogenen stöchiometrischen Überschusses an Phosgen in einer Mischzone und Führen des so erhaltenen gasförmigen Reaktionsgemisches durch eine Reaktionszone zur Umsetzung des primären Amins mit Phosgen unter Erhalt eines gasförmigen Reaktionsproduktgemisches;
(iv) Abkühlen des nach Durchlaufen der Reaktionszone aus Schritt (iii) erhaltenen gasförmigen Reaktionsproduktgemisches durch Inkontaktbringen mit mindestens einem Strom einer Quenchflüssigkeit in einer Quenchzone, wobei die insgesamt eingesetzte Quenchflüssigkeit, bezogen auf ihre Gesamtmasse, organische Lösungsmittel in einem Anteil von maximal 50,0 Massen-%, bevorzugt maximal 20,0 Massen-%, besonders bevorzugt maximal 10,0 Massen-%, ganz besonders bevorzugt 1,0 Massen-%, umfasst, wobei der Rest zu 100 Massen-% aus mindestens dem herzustellenden Isocyanat besteht, unter Erhalt eines Gemisches aus Reaktionsproduktgemisch und Quenchflüssigkeit;
(v) Führen des in Schritt (iv) erhaltenen Gemisches aus Reaktionsproduktgemisch und Quenchflüssigkeit in eine Sammelzone zur Trennung von flüssiger und gasförmiger Phase unter Erhalt einer flüssigen, Isocyanat und gegebenenfalls Lösungsmittel umfassenden Phase und einer gasförmigen, Chlorwasserstoff, nicht umgesetztes Phosgen, nicht verflüssigtes Isocyanat und gegebenenfalls verdampftes Lösungsmittel umfassenden Phase, wobei ein flüssiger Lösungsmittelstrom umfassend mehr als 50,0 Massen-%, insbesondere mindestens 51,0 Massen-%, bevorzugt mindestens 80,0 Massen-%, besonders bevorzugt mindestens 90,0 Massen-%, ganz besonders bevorzugt mindestens 99,0 Massen-%, an organischen Lösungsmitteln, bezogen auf die Gesamtmasse des flüssigen Lösungsmittelstroms, in die Sammelzone und/oder in einen strömungstechnisch nach der Sammelzone angeordneten Sammeltank für die in der Sammelzone erhaltene flüssige Phase eingeleitet wird.

Vollkommen überraschend wurde gefunden, dass - im Vergleich mit dem in FIG. 1 exemplifizierten Stand der Technik - ein Verzicht auf die obere, überwiegend Lösungsmittel enthaltende Quenchestufe in Verbindung mit einem Einleiten eines Lösungsmittelstroms *in die Sammelzone unterhalb der Quenchzone oder in einen strömungstechnisch nachgelagerten Sammeltank für die in der Sammelzone erhaltene flüssige Phase* dazu führt, dass es in der Quenchzone zu weniger Ablagerungen kommt, ohne dass die Qualität des Produkts darunter leidet und ohne dass sich die Verarbeitbarkeit des in der Sammelzone bzw. im Sammeltank anfallenden flüssigen Rohproduktes (etwa infolge eines unerwünschten starken Viskositätsanstiegs) verschlechtert, sowie dazu führt, dass die insgesamt eingesetzte Lösungsmittelmenge reduziert werden kann.

Erfindungsgemäß weist die in Schritt (iv) *"insgesamt eingesetzte Quenchflüssigkeit, bezogen auf ihre Gesamtmasse"* einen Anteil an organischen Lösungsmitteln von *"maximal 50,0 Massen-%, bevorzugt maximal 20,0 Massen-%, besonders bevorzugt maximal 10,0 Massen-%, ganz besonders bevorzugt 1,0,Massen-%"* auf. Wird in Schritt (iv) genau ein Strom an Quenchflüssigkeit eingesetzt, so muss dieser Strom diesen Anforderungen an den maximalen Lösungsmittelanteil genügen. Werden in Schritt (iv) zwei oder mehr Ströme an Quenchflüssigkeit aus unterschiedlichen Quellen mit unterschiedlicher Zusammensetzung eingesetzt, so muss der über alle in Schritt (iv) eingesetzten Ströme an Quenchflüssigkeit gemittelte Wert des Lösungsmittelanteils (also der *auf die Gesamtmasse der insgesamt eingesetzten Quenchflüssigkeit bezogene Anteil)* diesen Anforderungen genügen (bevorzugt genügt *jeder einzelne* in Schritt (iv) eingesetzte Strom an Quenchflüssigkeit den vorgenannten Anforderungen an den maximalen Lösungsmittelgehalt und besteht ansonsten aus mindestens dem herzustellenden Isocyanat). *"Organische Lösungsmittel"* (Lösungsmittel im Sinne der Erfindung sind stets *organische* Lösungsmittel) sind dabei dem Prozess zugesetzte, bei Normalbedingungen (20 °C, 1013 mbar) flüssige organische Verdünnungsmittel, die keine gegenüber Phosgen reaktive Gruppen aufweisen und in denen das herzustellende Isocyanat bei Normalbedingungen (20 °C, 1013 mbar) löslich ist, die jedoch selbstverständlich von diesem verschieden sind. Die dem Prozess zugesetzte Menge an solchen Lösungsmitteln ist bekannt; daraus und aus den ebenfalls bekannten Randbedingungen (Ort der Zugabe, Temperaturen und Drücke, Mengenströme der übrigen Einsatzstoffe) ist der Lösungsmittelanteil in der in Schritt (iv) insgesamt eingesetzten Quenchflüssigkeit für den Fachmann leicht zu ermitteln. Der nicht auf solche Lösungsmittel zurückgehende Anteil der insgesamt eingesetzten Quenchflüssigkeit besteht mindestens aus dem herzustellenden Isocyanat.

In bestimmten Varianten der Erfindung ist der Sammelzone *"strömungstechnisch"* ein *"Sammeltank für die in der Sammelzone erhaltene flüssige Phase"* nachgeschaltet, d. h. dass die flüssige Phase aus der Sammelzone - insofern sie nicht als Quenchflüssigkeit in Schritt (iv) rezykliert wird, wie in einigen weiter unten noch näher erläuterten Ausführungsformen vorgesehen - in diesen Sammeltank fließt. Verfügt eine Produktionsanlage über mehrere parallel geschaltete unabhängig voneinander regelbare Reaktionsstraßen (auch als *Reaktionsstränge* bezeichnet), so kann in einem solchen Sammeltank in vorteilhafter Weise das flüssige Rohprodukt aus allen Reaktionsstraßen gesammelt und anschließend einer gemeinsamen Aufarbeitung (insbesondere Destillation) zugeführt werden.

Die in Schritt (v) erhaltene *"flüssige, Isocyanat und gegebenenfalls Lösungsmittel umfassende Phase"* ist die bei der Phasentrennung in der Sammelzone anfallende flüssige Phase. Diese flüssige Phase enthält in einer **ersten Variante der Erfindung** (nämlich bei Einleitung des flüssigen Lösungsmittelstroms *nur in die Sammelzone*) stets Lösungsmittel, nämlich mindestens das Lösungsmittel aus dem auf diese Weise zugegebenen flüssigen Lösungsmittelstrom. In einer **zweiten Variante der Erfindung** (nämlich bei Einleitung des flüssigen Lösungsmittelstroms *nur in den Sammeltank*) enthält diese flüssige Phase nicht notwendigerweise Lösungsmittel, da der Lösungsmittelanteil in der Quenchflüssigkeit aus Schritt (iv) auch null sein kann. In einer **dritten Variante der Erfindung** wird ein flüssiger Lösungsmittelstrom in die Sammelzone *und* in den Sammeltank geleitet; in dieser Variante enthält die in die Sammelzone anfallende flüssige Phase auch Lösungsmittel. Die in Schritt (v) erhaltene *"gasförmige, Chlorwasserstoff, nicht umgesetztes Phosgen, nicht verflüssigtes Isocyanat und gegebenenfalls verdampftes Lösungsmittel"* umfassende Phase ist die bei der Phasentrennung in der Sammelzone anfallende gasförmige Phase.

Es folgt zunächst eine Kurzzusammenfassung verschiedener möglicher **Ausführungsformen der Erfindung:**
In einer **ersten Ausführungsform** der Erfindung, die mit allen anderen Ausführungsformen kombiniert werden kann, wird Schritt (iii) adiabatisch durchgeführt, wobei Zusammensetzung und Temperatur des gasförmigen Stroms des primären Amins in Schritt (i) und des Phosgenstroms in Schritt (ii) jeweils so gewählt werden, dass sich in Schritt (iii) in der Mischzone und in der Reaktionszone eine Temperatur im Bereich von 250 °C bis 450 °C, bevorzugt im Bereich von 270 °C bis 425 °C, besonders bevorzugt im Bereich von 280 °C bis 420 °C einstellt.

In einer **zweiten Ausführungsform** der Erfindung, die mit allen anderen Ausführungsformen kombiniert werden kann, wird die Temperatur der in Schritt (iv) eingesetzten Quenchflüssigkeit auf einen Wert im Bereich von 50 °C bis 250 °C, bevorzugt 100 °C bis 200 °C, besonders bevorzugt 120 °C bis 190 °C, eingestellt.

In einer **dritten Ausführungsform** der Erfindung, die mit allen anderen Ausführungsformen kombiniert werden kann, wird der Mengenstrom der in Schritt (iv) eingesetzten Quenchflüssigkeit so gewählt, dass dieser das 2-fache bis 60-fache, bevorzugt das 10-fache bis 50-fache, besonders bevorzugt das 20-fache bis 40-fache des Mengenstroms an gasförmigem Strom des primären Amins aus Schritt (i) beträgt.

In einer **vierten Ausführungsform** der Erfindung, die mit allen anderen Ausführungsformen kombiniert werden kann, wird die Temperatur des in Schritt (v) in die Sammelzone oder den Sammeltank geleitetne flüssige Lösungsmittelstroms auf einen Wert im Bereich von 120 °C bis 200 °C, bevorzugt von 180 °C bis 190 °C eingestellt.

In einer **fünften Ausführungsform** der Erfindung, die mit allen anderen Ausführungsformen außer der nachfolgend beschriebenen sechsten kombiniert werden kann, wird der flüssige Lösungsmittelstrom in Schritt (v) *nur* in die Sammelzone geleitet **(erste Variante** der Erfindung), wobei der flüssige Lösungsmittelstrom in einem solchen Mengenstrom in die Sammelzone geleitet wird, dass sein Anteil an der Gesamtmasse der in der Sammelzone erhaltenen flüssigen, Isocyanat und Lösungsmittel umfassenden Phase im Bereich von 20 Massen-% bis 80 Massen-%, bevorzugt im Bereich von 40 Massen-% bis 60 Massen-%, liegt.

In einer **sechsten Ausführungsform** der Erfindung, die mit allen anderen Ausführungsformen außer der fünften kombiniert werden kann, wird der flüssige Lösungsmittelstrom in Schritt (v) entweder (a) in den Sammeltank **(zweite Variante** der Erfindung) oder (b) in den Sammeltank *und* in die Sammelzone **(dritte Variante** der Erfindung) geleitet, wobei der Mengenstrom des flüssigen Lösungsmittelstroms jeweils so gewählt wird, dass sein Anteil an der Gesamtmasse der im Sammeltank erhaltenen flüssigen, Isocyanat und Lösungsmittel umfassenden Phase im Bereich von 20 Massen-% bis 80 Massen-%, bevorzugt im Bereich von 40 Massen-% bis 60 Massen-%, liegt.

In einer **siebten Ausführungsform** der Erfindung, die mit allen anderen Ausführungsformen kombiniert werden kann, erfolgt das Inkontaktbringen des gasförmigen Reaktionsproduktgemisches mit der Quenchflüssigkeit in Schritt (iv) durch Eindüsen der Quenchflüssigkeit in das gasförmige Reaktionsproduktgemisch.

In einer **achten Ausführungsform** der Erfindung, die eine besondere Ausgestaltung der siebten Ausführungsform ist, erfolgt das Inkontaktbringen des gasförmigen Reaktionsproduktgemisches mit der Quenchflüssigkeit in Schritt (iv) einstufig.

In einer **neunten Ausführungsform** der Erfindung, die mit allen anderen Ausführungsformen kombiniert werden kann, wird die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase in einem Schritt (viii-1) durch indirekte Abkühlung unter Erhalt eines flüssigen, Isocyanat und gegebenenfalls Lösungsmittel umfassenden Stroms und eines gasförmigen, Chlorwasserstoff, Phosgen, gegebenenfalls nicht verflüssigtes Isocyanat und gegebenenfalls nicht verflüssigtes Lösungsmittel umfassenden Stroms kondensiert, insbesondere nur teilweise kondensiert.

In einer **zehnten Ausführungsform** der Erfindung, die eine besondere Ausgestaltung der neunten Ausführungsform ist, wird der in Schritt (viii-1) erhaltene gasförmige, Chlorwasserstoff, Phosgen, gegebenenfalls nicht verflüssigtes Isocyanat und gegebenenfalls nicht verflüssigtes Lösungsmittel umfassende Strom in einem Schritt (viii-2) in eine Auswaschkolonne geleitet und dort mit mindestens einem flüssigen Lösungsmittelstrom als Auswaschflüssigkeit in Kontakt gebracht, wobei ein flüssiger, Lösungsmittel und Isocyanat umfassender Strom und ein gasförmiger, Chlorwasserstoff und Phosgen umfassender Strom erhalten werden.

In einer **elften Ausführungsform** der Erfindung, die mit allen anderen Ausführungsformen außer der neunten und zehnten kombiniert werden kann, wird die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase in einem Schritt (viii-2) in eine Auswaschkolonne geleitet (d. h. *ohne* vorherige Kondensation in Schritt (viii-1)) und dort mit mindestens einem flüssigen Lösungsmittelstrom als Auswaschflüssigkeit in Kontakt gebracht, wobei ein flüssiger, Lösungsmittel und Isocyanat umfassender Strom und ein gasförmiger, Chlorwasserstoff und Phosgen umfassender Strom erhalten werden.

In einer **zwölften Ausführungsform** der Erfindung, die eine besondere Ausgestaltung der neunten und zehnten Ausführungsform ist, umfasst die in der Sammelzone erhaltene gasförmige, Chlorwasserstoff, nicht umgesetztes Phosgen und nicht verflüssigtes Isocyanat umfassende Phase verdampftes Lösungsmittel, wobei der in Schritt (v) in die Sammelzone oder den Sammeltank geleitete flüssige Lösungsmittelstrom den in Schritt (viii-1) erhaltenen flüssigen, Isocyanat und Lösungsmittel umfassenden Strom umfasst.

In einer **dreizehnten Ausführungsform** der Erfindung, die eine besondere Ausgestaltung der zehnten, elften und zwölften Ausführungsform ist, wird Schritt (viii-2) in jedem Fall durchgeführt, wobei der in Schritt (v) in die Sammelzone oder den Sammeltank geleitete flüssige Lösungsmittelstrom den in Schritt (viii-2) erhaltenen flüssigen, Lösungsmittel und Isocyanat umfassenden Strom umfasst.

In einer **vierzehnten Ausführungsform** der Erfindung, die eine besondere Ausgestaltung der zwölften und dreizehnten Ausführungsform ist, umfasst der Lösungsmittelstrom aus Schritt (v) keine weiteren Bestandteile außer den genannten flüssigen Isocyanat und Lösungsmittel umfassenden Strömen aus Schritt (viii-1) und/oder Schritt (viii-2).

In einer **fünfzehnten Ausführungsform** der Erfindung, die mit allen anderen Ausführungsformen kombiniert werden kann, vorteilhafterweise insbesondere mit der zwölften, dreizehnten oder vierzehnten Ausführungsform, wird in Schritt (v) der flüssige Lösungsmittelstrom zumindest zeitweise in die Sammelzone *und* in den Sammeltank geleitet, wobei die in die Sammelzone und den Sammeltank führenden Leitungen für diesen Lösungsmittelstrom unabhängig voneinander regelbar sind (d. h. der Massenstrom durch eine dieser Leitungen ist unabhängig von der anderen Leitung regulierbar).

In einer **sechszehnten Ausführungsform** der Erfindung, die mit allen anderen Ausführungsformen kombiniert werden kann, wird ein Teil der in Schritt (v) in der Sammelzone erhaltenen flüssigen, Isocyanat und gegebenenfalls Lösungsmittel umfassenden Phase in einem Schritt (vi), bevorzugt durch indirekte Kühlung, abgekühlt aber ansonsten nicht in seiner Zusammensetzung verändert und in die Quenchzone aus Schritt (iv) rezykliert wobei insbesondere keine weiteren Ströme an Quenchflüssigkeit eingesetzt werden.

In einer **siebzehnten Ausführungsform** der Erfindung, die mit allen anderen Ausführungsformen kombiniert werden kann, insbesondere vorteilhafterweise mit der sechszehnten Ausführungsform, wird mindestens ein Teil der in Schritt (v) in der Sammelzone erhaltenen flüssigen, Isocyanat und gegebenenfalls Lösungsmittel umfassenden Phase, insbesondere der nicht in der sechszehnten Ausführungsform in Schritt (vi) in die Quenchzone aus Schritt (iv) rezyklierte Teil, in einem Schritt (vii) zu Rein-Isocyanat aufgearbeitet.

In einer **achtzehnten Ausführungsform** der Erfindung, die mit allen anderen Ausführungsformen kombiniert werden kann, enthält der in Schritt (v) in die Sammelzone oder den Sammeltank geleitete flüssige Lösungsmittelstrom ausschließlich Lösungsmittel ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten Lösungsmittel enthält, wobei
- das in der in Schritt (iv) eingesetzten Quenchflüssigkeit optional vorhandene Lösungsmittel, sofern vorhanden, und
- das in dem in Schritt (viii-2), sofern durchgeführt, als Auswaschflüssigkeit eingesetzten flüssigen Lösungsmittelstrom enthaltende Lösungsmittel
jeweils das gleiche ist wie das in dem in Schritt (v) in die Sammelzone oder den Sammeltank geleiteten flüssigen Lösungsmittelstrom enthaltende Lösungsmittel.

In einer **neunzehnten Ausführungsform** der Erfindung, die mit allen anderen Ausführungsformen kombiniert werden kann, wird das primäre Amin ausgewählt aus der Gruppe bestehend aus Toluylendiamin, Diphenylmethandiamin, Xylylendiamin, 1,5-Pentandiamin, 1,6-Hexamethylendiamin, Isophorondiamin, Diaminodicyclohexylmethan und Mischungen der vorgenannten Verbindungen.

In einer **zwanzigsten Ausführungsform** der Erfindung, die mit allen anderen Ausführungsformen kombiniert werden kann, wird in allen Schritten des Verfahrens, in denen ein organisches Lösungsmittel eingesetzt wird, das gleiche organische Lösungsmittel eingesetzt, und dieses organische Lösungsmittel wird ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel.

In einer **einundzwanzigsten Ausführungsform** der Erfindung, die eine besondere Ausgestaltung der zwanzigsten Ausführungsform ist, ist das organische Lösungsmittel ortho-Dichlorbenzol.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

In **Schritt** (i) wird ein Strom eines primäre Amins in gasförmiger Form bereitgestellt.

Geeignete *primäre Amine* für die Durchführung dieses Schrittes sind solche, die sich unzersetzt verdampfen lassen, insbesondere Toluylendiamin (nachfolgend TDA, insbesondere *meta-*TDA)*,* Diphenylmethandiamin (oft auch Methylendiphenyldiamin genannt, nachfolgend MDA), Xylylendiamin (XDA, insbesondere *meta-*XDA), 1,5-Pentandiamin (nachfolgend PDA), 1,6-Hexamethylendiamin (nachfolgend HDA), Isophorondiamin (nachfolgend IDPA) und Diaminodicyclohexylmethan (nachfolgend H12-MDA). Sofern die genannten Amine in verschiedenen isomeren Formen vorliegen können, ohne dass diese angegeben sind, sind alle Isomerenverteilungen umfasst. Prinzipiell können auch Gemische der vorgenannten Amine umgesetzt werden, obwohl dies im Allgemeinen nicht bevorzugt ist.

Besonders bevorzugt ist TDA. Üblicherweise umfasst TDA, welches bevorzugt eingesetzt wird, 78,0 Massen-% bis 82,0 Massen-% 2,4-TDA und 18,0 Massen-% bis 22,0 Massen-% 2,6-TDA, bezogen auf die Gesamtmasse der 2,4- und 2,6-TDA-Isomeren. Bezogen auf die Gesamtmasse des TDA machen dabei die 2,4- und 2,6-TDA-Isomeren bevorzugt in Summe von 95,0 Massen-% bis 100 Massen-%, besonders bevorzugt von 98,0 Massen-% bis 100 Massen-%, aus. Bevorzugt beträgt der Gehalt an TDA-Isomeren mit zueinander ortho-ständigen NH₂-Gruppen im einzusetzenden TDA weniger als 0,2 Massen-%, bezogen auf die Gesamtmasse des einzusetzenden TDA. Verfahren zur Bereitstellung von TDA mit den genannten Anforderungen sind dem Fachmann bekannt.

Methoden zur Bereitstellung eines gasförmigen Aminstroms für die Durchführung von Schritt (i) sind dem Fachmann grundsätzlich bekannt. Im Folgenden werden bevorzugte Ausführungsformen geschildert.

Die Überführung des Amins in die Gasphase kann in allen aus dem Stand der Technik bekannten Verdampfungsapparaturen erfolgen, insbesondere in einem Fallfilmverdampfer. Bevorzugt werden solche Verdampfungsapparaturen eingesetzt, bei denen ein kleiner Arbeitsinhalt mit einer hohen Umwälzleistung über einen Fallfilmverdampfer geführt wird.

In einer bevorzugten Ausführungsform des erfindungsgemäßen Verfahrens werden Verdampfungsapparaturen eingesetzt, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Aminen wird z. B. in EP 1 754 698 A2 offenbart. Bevorzugt werden in dem erfindungsgemäßen Verfahren die in den Absätzen [0007] bis [0008] und [0017] bis [0039] der in EP 1 754 698 A2 offenbarten Apparate eingesetzt.

Zur Minimierung der thermischen Belastung des Amins ist es unabhängig von der genauen Ausgestaltung der Verdampfungsapparatur bevorzugt, den Verdampfungsvorgang durch Zuspeisung eines Inertgases wie N₂, He, Ar oder der Dämpfe eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel, zu unterstützen. Wird das Amin auf diese Weise verdünnt, so ist der Einsatz eines Inertgases ausgewählt aus der Gruppe bestehend aus N₂, He, Ar und Mischungen derselben als Verdünnungsmittel unter Verzicht auf die Verwendung von Dämpfen eines inerten Lösungsmittels bevorzugt.

Weiterhin erfolgt die Verdampfung - und erforderlichenfalls Überhitzung - des Ausgangsamins (insbesondere auf eine Temperatur im Bereich von 200 °C bis 430 °C, bevorzugt 250 °C bis 420 °C, besonders bevorzugt 250 °C bis 400 °C) bevorzugt mehrstufig, um unverdampfte Tröpfchen im gasförmigen Aminstrom zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind dem Fachmann bekannt. Nach Verlassen des in Strömungsrichtung letzten Überhitzers wird das auf seine Solltemperatur vorgeheizte gasförmige Amin mit einer mittleren Verweilzeit von bevorzugt 0,01 s bis 60 s, ganz besonders bevorzugt von 0,01 s bis 30 s, insbesondere bevorzugt 0,01 s bis 15 s, der der Vermischung und Umsetzung in Schritt (iii) zugeführt. Unabhängig von der Ausgestaltung der Zuführung des Amins im Detail wird bevorzugt über technische Maßnahmen, z. B. eine ausreichende Isolierung zur Vermeidung von Abstrahlungsverlusten, der Gefahr einer erneuten Tröpfchenbildung begegnet.

In **Schritt** (ii) wird ein gasförmiger Phosgenstrom bereitgestellt.

Bei dem erfindungsgemäßen Verfahren wird Phosgen im Überschuss bezüglich der umzusetzenden Aminogruppen eingesetzt. Bevorzugt wird ein molares Verhältnis von Phosgen zu Amingruppen von 1,1 : 1 bis 20: 1, besonders bevorzugt 1,2 : 1 bis 5,0 : 1 eingestellt. Auch das Phosgen wird bevorzugt, wie zuvor für das Amin beschrieben, auf eine Temperatur im Bereich von 200 °C bis 430 °C, bevorzugt 250 °C bis 420 °C, besonders bevorzugt 250 °C bis 400 °C, erhitzt und gegebenenfalls verdünnt mit einem Inertgas wie N₂, He, Ar oder mit den Dämpfen eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel der Vermischung und Umsetzung in Schritt (iii) zugeführt. Wird das Phosgen verdünnt, so ist der Einsatz eines Inertgases ausgewählt aus der Gruppe bestehend aus N₂, He, Ar und Mischungen derselben als Verdünnungsmittel unter Verzicht auf die Verwendung von Dämpfen eines inerten Lösungsmittels bevorzugt.

In **Schritt (iii)** werden die Reaktionspartner Amin und Phosgen vermischt und umgesetzt.

Die getrennt aufgeheizten Reaktionspartner Amin und Phosgen werden bevorzugt über eine Düsenanordnung der Vermischung und Umsetzung in Schritt (iii) zugeführt. Die Düsenanordnung zur Einführung der Eduktgasströme Amin und Phosgen kann auf verschiedene dem Fachmann bekannte Arten ausgestaltet sein; Beispiele finden sich z. B. in EP 2 199 277 B1, Abschnitt [0017] bis [0019], EP 1 449 826 B1, Abschnitt [0011] bis [0012], EP 1 362 847 B1, Abschnitt [0011] bis [0012], EP 1 526 129 B1, Abschnitt [0009] bis [0011] und EP 1 555 258 B1, Abschnitt [0008] bis [0011].

Neben der bereits erwähnten Möglichkeit, den gasförmigen Strom des primären Amins und den gasförmigen Phosgenstrom zu verdünnen, kann auch ein separater Verdünnungsgasstrom (ein Inertgas wie N₂, He, Ar oder die Dämpfe eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel) direkt in die Vermischung in Schritt (iii) gefahren werden. In diesem Fall wird dieser Verdünnungsgasstrom bevorzugt auf eine Temperatur im Bereich von 100 °C bis 500 °C, bevorzugt 150 °C bis 450 °C, besonders bevorzugt 150 °C bis 400 °C erhitzt. Wird der Mischzone aus Schritt (iii) ein solcher Verdünnungsgasstrom zugeführt, so ist der Einsatz eines Inertgases ausgewählt aus der Gruppe bestehend aus N₂, He, Ar und Mischungen derselben als Verdünnungsgas unter Verzicht auf die Verwendung von Dämpfen eines inerten Lösungsmittels bevorzugt.

Die Umsetzung in der Reaktionszone erfolgt bevorzugt adiabatisch. Adiabatische Umsetzung bedeutet, dass auf eine gezielte Abfuhr der entstandenen Reaktionswärme durch ein Wärmeträgermedium verzichtet wird. Daher spiegelt sich die Reaktionsenthalpie - abgesehen von unvermeidbaren Wärmeverlusten - quantitativ in der Temperaturdifferenz von Produkt- und Eduktgasstrom wider. Insbesondere betrifft die Erfindung auch ein Verfahren, bei welchem Schritt (iii) adiabatisch durchgeführt wird und wobei Zusammensetzung und Temperatur des gasförmigen Stroms des primären Amins in Schritt (i) und des Phosgenstroms in Schritt (ii) jeweils so gewählt werden, dass sich in Schritt (iii) in der Mischzone und in der Reaktionszone eine Temperatur im Bereich von 250 °C bis 450 °C, bevorzugt im Bereich von 270 °C bis 425 °C, besonders bevorzugt im Bereich von 280 °C bis 420 °C, einstellt. Damit ist gemeint, dass die Temperatur an jedem Punkt in der Mischzone und der Reaktionszone in diesem Bereich liegt. Mischzone und Reaktionszone sind dabei bevorzugt in einer gemeinsamen technischen Vorrichtung zur Durchführung chemischer Reaktionen angeordnet, dem *Reaktor.* Bei dieser Anordnung gehen im Allgemeinen Misch- und Reaktionszone fließend ineinander über, ohne dass - wie bei Einsatz einer separaten Mischapparatur, was grundsätzlich auch möglich ist - eine strenge Abgrenzung beider möglich wäre. Die Reaktionszone nach Vermischung der Reaktanden dient zur Bereitstellung von Verweilzeitraum, um möglichst vollständigen Umsatz sicher zu stellen.

Der Reaktor hat bevorzugt im Bereich der Mischzone und im Bereich der Reaktionszone einen runden (insbesondere kreissymmetrischen) Querschnitt. Dabei kann der ganze Reaktor zylinderförmig sein. Es ist jedoch auch möglich, dass sich der Querschnitt verändert wie z. B. in EP 1275639 B1, Abschnitt [0009], EP 1275 640 A1, Abschnitt [0014], EP 1 403 248 B1, Abschnitt [0014] bis [0015], EP 193 5876 A1, Abschnitt [0012] bis [0016] und EP 2 196 455 B1, Abschnitte [0015] bis [0026] und [0027] bis [0030] beschrieben. Weitere Details zum Bau geeigneter Phosgenierungsreaktoren sind dem Fachmann bekannt.

In der Reaktionszone werden Amin und Phosgen rasch zum korrespondierenden Isocyanat umgesetzt, wie beschrieben bevorzugt adiabatisch. Die Reaktion wird bevorzugt so geführt, dass das Amin vor Eintritt in die weiter unten näher beschriebene Quenchzone vollständig umgesetzt ist. In einer bevorzugten Ausführungsform des erfindungsgemäßen Verfahrens beträgt die Durchsatzkapazität des eingesetzten Reaktors unter Reaktionsbedingungen > 1,0 t Amin / h, bevorzugt 2,0 bis 50 t Amin / h, besonders bevorzugt 5,0 bis 15 t Amin / h. Besonders bevorzugt gelten diese Werte für Toluylendiamin, 1,6-Diaminohexan und für Isophorondiamin. Unter Durchsatzkapazität ist dabei zu verstehen, dass in dem Reaktor die genannte Durchsatzkapazität an Amin pro Stunde umgesetzt werden kann.

Bevorzugt strömen die Edukt- und Produktgase ohne wesentliche Rückvermischung durch den Reaktor. Dies wird durch ein Druckgefälle über die Misch- und Reaktionszone, bevorzugt über die Mischzone, Reaktionszone und die sich anschließende Quenchzone, sichergestellt. Bevorzugt besteht das Druckgefälle zwischen dem Beginn der Mischzone einerseits und dem Ausgang aus der Quenchzone andererseits. Bevorzugt liegt der absolute Druck am Beginn der Mischzone bei 200 mbar bis 3000 mbar und hinter der Quenchzone bei 150 mbar bis 2500 mbar. Wesentlich ist hierbei jedoch lediglich die Aufrechterhaltung eines Differenzdrucks über die Reaktionszone, bevorzugt über die Reaktionszone und die Quenchzone von bevorzugt mindestens 40 mbar zwecks Gewährleistung der genannten gerichteten Strömung und einer guten Vermischung der Edukte.

In **Schritt (iv)** erfolgt die rasche Abkühlung und weitgehende Verflüssigung des gebildeten Isocyanats ("Quenche").

Die erfindungsgemäße Forderung, dass *"die insgesamt eingesetzte Quenchflüssigkeit, bezogen auf ihre Gesamtmasse, organische Lösungsmittel in einem Anteil von maximal 50,0 Massen-%, bevorzugt maximal 20,0 Massen-%, besonders bevorzugt maximal 10,0 Massen-%, ganz besonders bevorzugt 1,0 Massen-%, umfasst, wobei der Rest zu 100 Massen-% aus mindestens dem herzustellenden Isocyanat besteht"* bedeutet, dass im Rahmen der vorliegenden Erfindung der im Stand der Technik übliche Einsatz von Quencheflüssigkeit mit einem hohen Lösungsmittelgehalt (mehr als 50,0 Massen-%, insbesondere 51,0 Massen-% oder mehr - solche Ströme sind in der Terminologie der vorliegenden Erfindung als *"Lösungsmittelströme"* aufzufassen) *nicht angewandt wird.* Sofern die in Schritt (iv) eingesetzte Quenchflüssigkeit ein organisches Lösungsmittel enthält, ist dieses bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel.

Nach der erfolgten Phosgenierungsreaktion in der Reaktionszone wird das gasförmige Reaktionsproduktgemisch, das wenigstens das herzustellende Isocyanat, Chlorwasserstoff und nicht umgesetztes (da überstöchiometrisch eingesetzt) Phosgen umfasst, in die *Quenchzone* geleitet, wo das gebildete Isocyanat durch Inkontaktbringen mit, d. h. insbesondere durch Eindüsen von, Quenchflüssigkeit abgekühlt und so (weitgehend) kondensiert wird. Möglichkeiten für den Aufbau und den Betrieb einer Quenchzone sind im Prinzip aus dem Stand der Technik bekannt. Sofern die erfindungsgemäßen Anforderungen im Hinblick auf die Zusammensetzung der Quenchflüssigkeit eingehalten werden, können die Apparate und Methoden des Standes der Technik eingesetzt werden. Mögliche Ausgestaltungen der Quenchzone sind beispielsweise in EP 1 403 248 A1 und EP 1 935 875 A1 offenbart. Einhaltung der erfindungsgemäßen Anforderungen im Hinblick auf die Zusammensetzung der Quenchflüssigkeit bedeutet, dass die im gesamten Quencheschritt (iv) insgesamt eingesetzte Quenchflüssigkeit 0,0 Massen-% bis 50,0 Massen-% an organischen Lösungsmitteln, bevorzugt 0,0 Massen-% bis 20,0 Massen-% an organischen Lösungsmitteln, besonders bevorzugt 0,0 Massen-% bis 1,0 Massen-% an organischen Lösungsmitteln enthält, jeweils bezogen auf die Gesamtmasse der insgesamt eingesetzten Quenchflüssigkeit. Werden mehrere Ströme an Quenchflüssigkeit eingesetzt, so gelten die zuvor genannten Zusammensetzungsbereiche mindestens für den über alle eingesetzten Ströme an Quenchflüssigkeit gemittelten Massenanteil an organischen Lösungsmitteln, bevorzugt jedoch für jeden einzelnen Strom an Quenchflüssigkeit. In einer weiter unten noch näher erläuterten Ausführungsform wird ein Teil der in der Sammelzone in Schritt (v) erhaltenen flüssigen Phase nach Abkühlung in die Quenche aus Schritt (iv) rezykliert (d. h. als Quenchflüssigkeit eingesetzt). Wird diese Ausführungsform in der ersten oder dritten Variante der Erfindung eingesetzt (Einleitung eines flüssigen Lösungsmittelstroms in Schritt (v) *in die Sammelzone*)*,* so enthält die in Schritt (iv) eingesetzte Quenchflüssigkeit zwangsläufig Lösungsmittel. Der Anteil an Lösungsmitteln in dem auf diese Weise rezyklierten Teilstrom der in Schritt (v) erhaltenen flüssigen Phase beträgt bevorzugt 2,0 Massen-% bis 10,0 Massen-%, besonders bevorzugt 5,0 Massen-% bis 10,0 Massen-%, jeweils bezogen auf die Gesamtmasse des rezyklierten Teilstroms.

In einer bevorzugten Ausführungsform wird Schritt (iv) so ausgestaltet, dass die Quenchflüssigkeit an nur einer Position der Quenchzone in das Reaktionsproduktgemisch eingedüst wird (einstufige Quenche). Mit "*an nur einer Position* " ist dabei bei einer Quenchzone in einem aufrecht stehenden Reaktor mit rundem (insbesondere kreissymmetrischen) Querschnitt eine einzige Position entlang der Längsrichtung des Reaktors gemeint. Dabei kann die Quenchflüssigkeit durchaus mithilfe mehrerer Düsen *entlang des Querschnitts* der Quenchzone verteilt werden. Dies geschieht jedoch an derselben Position entlang der Längsrichtung des Reaktors, im Unterschied zur in FIG. 1 gezeigten Verfahrensweise mit Zufuhr von Quenchflüssigkeit an *zwei unterschiedlichen* Positionen (siehe 116 und 105) entlang der Längsrichtung des Reaktors.

Die Temperatur der in Schritt (iv) eingesetzten Quenchflüssigkeit wird bevorzugt so gewählt, dass sie einerseits hoch genug ist, um das dem Isocyanat entsprechende Carbamoylchlorid in Isocyanat und Chlorwasserstoff zurückzuspalten. (Es ist zwar keineswegs sicher, ob das aus der Flüssigphasenphosgenierung bekannte Zwischenprodukt Carbamoylclorid auch in der Gashasenphosgenierung gebildet wird. Da es jedoch unabhängig davon denkbar ist, dass in der Quenche *verflüssigtes* Isocyanat mit dem vorhandenem Chlorwasserstoffgas teilweise zum Carbamoylchlorid reagiert, sollte die Temperatur der Quenchflüssigkeit hoch genug sein, um diese Reaktion zurückzudrängen.) Andererseits sollen Isocyanat und gegebenenfalls das in dem gasförmigen Aminstrom und/oder gasförmigen Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und gegebenenfalls als Verdünnungsmittel mit verwendetes Inertgas die Quenchzone weitestgehend nicht kondensiert bzw. nicht gelöst durchlaufen, sodass die Temperatur der Quenchflüssigkeit auch nicht zu hoch gewählt werden darf. Zur selektiven Gewinnung des Isocyanats aus dem gasförmigen Reaktionsgemisch besonders gut geeignet sind insbesondere bei einer Temperatur von 50 °C bis 250 °C, bevorzugt 100 °C bis 200 °C, besonders bevorzugt 120 °C bis 190 °C, gehaltene Quenchflüssigkeiten (werden mehrere Ströme an Quenchflüssigkeit eingesetzt, so wird die Temperatur eines jeden Stroms an Quenchflüssigkeit in diesem Bereich gehalten). Dabei wird der Mengenstrom an in Schritt (iv) insgesamt eingesetzter Quenchflüssigkeit bevorzugt so gewählt, dass dieser das 2-fache bis 60-fache, besonders bevorzugt das 10-fache bis 50-fache, ganz besonders bevorzugt das 20-fache bis 40-fache des Mengenstroms an gasförmigem Strom des primären Amins aus Schritt (i) beträgt.

Es ist für den Fachmann aufgrund der physikalischen Daten bei gegebener Temperatur, Druck und Zusammensetzung einfach vorhersagbar, welcher Massenanteil des Isocyanats in der Quenche kondensiert bzw. diese nicht kondensiert durchläuft. Ebenso ist es einfach vorherzusagen, welcher Massenanteil des überschüssigen Phosgens, Chlorwasserstoffs gegebenenfalls Lösungsmittel und gegebenenfallsgegebenenfalls als Verdünnungsmittel verwendeten Inertgases die Quenche unkondensiert durchläuft bzw. sich in der Quenchflüssigkeit löst. Das so in der Quenchzone erhaltene Gemisch aus Reaktionsproduktgemisch und Quenchflüssigkeit enthält daher gasförmige Anteile und flüssige Anteile, ist also zweiphasig.

Bevorzugt sind Mischzone, Reaktionszone und Quenchzone in einer gemeinsamen Vorrichtung, dem Reaktor, angeordnet. Insbesondere bevorzugt hat der Reaktor im Bereich der Mischzone, Reaktionszone und Quenchzone einen runden (insbesondere kreissymmetrischen) Querschnitt und ist entweder von zylindrischer Form oder weist - wie oben beschrieben - Abschnitte unterschiedlichen Querschnitts auf, sodass der Reaktor im Bereich der Mischzone, Reaktionszone und Quenchzone aus Zylindern unterschiedlichen Durchmessers, die über kegelförmige Übergangsstücke verbunden sind, besteht. Der Reaktor steht dabei bevorzugt aufrecht, und die Eduktgasströme Amin und Phosgen (sowie die aus diesen gebildeten Zwischen- und Endprodukte) durchlaufen den Reaktor bevorzugt *von oben nach unten.* Mischzone, Reaktionszone und Quenchzone sind dann in dieser Reihenfolge von oben nach unten in dem Reaktor angeordnet. Bevorzugt ist auch die im Folgenden noch näher geschilderte Sammelzone Bestandteil des Reaktors und ist bei aufrechter Anordnung desselben insbesondere unterhalb der Quenchzone angeordnet. Auch die Sammelzone hat bevorzugt einen runden (insbesondere kreissymmetrischen) Querschnitt; andere Ausgestaltungen sind aber denkbar.

In **Schritt (v)** wird das in Schritt (iv) anfallende Gemisch aus Reaktionsproduktgemisch und Quenchflüssigkeit in eine *Sammelzone* zur Phasentrennung geführt.

In der zuvor geschilderten bevorzugten Ausführungsform der gemeinsamen Anordnung von Mischzone, Reaktionszone, Quenchzone und Sammelzone von oben nach unten in der genannten Reihenfolge in einem aufrecht stehenden Reaktor fließt das in Schritt (iv) anfallende Gemisch aus Reaktionsproduktgemisch und Quenchflüssigkeit schwerkraftbedingt (also "automatisch") in die Sammelzone. Bei anderer Anordnung der Sammelzone ist es unter Umständen erforderlich, das Gemisch aus Reaktionsproduktgemisch und Quenchflüssigkeit in die Sammelzone zu pumpen. In der Sammelzone findet eine Auftrennung des in Schritt (iv) erhaltenen Gemisches aus Reaktionsproduktgemisch und Quenchflüssigkeit in eine flüssige, Isocyanat und gegebenenfalls Lösungsmittel umfassende Phase und eine gasförmige, (mindestens) Chlorwasserstoff, nicht umgesetztes Phosgen und nicht verflüssigtes Isocyanat und gegebenenfalls verdampftes Lösungsmittel umfassende Phase statt. Die flüssige und gasförmige Phase werden der Sammelzone kontinuierlich entnommen.

Erfindungsgemäß wesentlich ist nun, dass *in diese Sammelzone* oder *in einen der Sammelzone strömungstechnisch nachgelagerten Sammeltank für die in der Sammelzone erhaltene flüssige Phase* zusätzlich ein flüssiger Lösungsmittelstrom umfassend mehr als 50,0 Massen-%, insbesondere mindestens 51,0 Massen-%, bevorzugt mindestens 80,0 Massen-%, besonders bevorzugt mindestens 90,0 Massen-%, ganz besonders bevorzugt mindestens 99,0 Massen-%, an organischen Lösungsmitteln, bezogen auf die Gesamtmasse des Lösungsmittelstroms, eingeleitet wird. Hierdurch kann die Viskosität des flüssigen Rohprodukts gezielt auf einen gewünschten, für die weitere Verarbeitung geeigneten, Wert eingestellt werden. Außerdem ist es hierdurch möglich, die Temperatur des Gemisches aus Reaktionsproduktgemisch und Quenchflüssigkeit weiter zu senken und auf diese Weise unerwünschte Nebenreaktionen zu minimieren. Bevorzugt wird das organische Lösungsmittel, das in den oben genannten Anteilen in dem flüssigen Lösungsmittelstrom enthalten ist, ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel. Die Temperatur des in diesem Schritt (v) in die Sammelzone oder in einen nachgelagerten Sammeltank geleiteten flüssigen Lösungsmittelstroms liegt bevorzugt im Bereich von 120 °C bis 200 °C, besonders bevorzugt im Bereich von 180 °C bis 190 °C. Der Mengenstrom des in der ersten Variante der Erfindung *in die Sammelzone* geleiteten flüssigen Lösungsmittelstroms wird bevorzugt so gewählt, dass sein Anteil an der Gesamtmasse der flüssigen Phase, die sich in der Sammelzone ausbildet und in diesem Fall neben Isocyanat stets auch Lösungsmittel enthält, im Bereich von 20 Massen-% bis 80 Massen-%, besonders bevorzugt im Bereich von 40 Massen-% bis 60 Massen-%, liegt. Wird der flüssige Lösungsmittelstrom in der zweiten Variante der Erfindung nur in den Sammeltank geleitet, so gelten die genannten Zahlenwerte entsprechend für die im Sammeltank vorhandene flüssige Phase, d. h. der auf die Gesamtmasse der flüssige Phase des Sammeltanks bezogene Lösungsmittelanteil in dieser flüssigen Phase liegt bevorzugt im Bereich von 20 Massen-% bis 80 Massen-%, besonders bevorzugt im Bereich von 40 Massen-% bis 60 Massen-%. In der dritten Variante der Erfindung wird wie in der zweiten Variante bevorzugt ein auf die Gesamtmasse der flüssigen Phase des Sammeltanks bezogener Lösungsmittelanteil in dieser flüssigen Phase im Bereich von 20 Massen-% bis 80 Massen-%, besonders bevorzugt im Bereich von 40 Massen-% bis 60 Massen-%, eingestellt. Die dritte Variante erlaubt es, den Lösungsmittelstrom, der bevorzugt aus dem weiter unten noch näher zu schildernden Schritt (viii) stammt (vgl. auch die oben genannte **fünfzehnte Ausführungsform),** entsprechend den in einer bestimmten Situation konkret vorliegenden Anforderungen auf die Sammelzone und den Sammeltank aufzuteilen. Wird beispielsweise angestrebt, das aus Schritt (iv) stammende Gemisch aus Reaktionsproduktgemisch und Quenchflüssigkeit besonders rasch noch weiter abzukühlen, so kann der in die Sammelzone geleitete Anteil des Lösungsmittelstroms zu Lasten des in den Sammeltank geleiteten Anteils erhöht werden. Zu diesem Zweck müssen selbstverständlich entsprechende mit Regelventilen versehene Leitungen vorhanden sein, wie weiter unten bei der Erläuterung von FIG. 3 noch näher beschrieben wird.

Die *"Gesamtmasse der flüssigen Phase"* meint dabei in allen zuvor geschilderten Varianten und deren Ausführungsformen die Masse der jeweiligen Flüssigphase inklusive der Masse des zugeführten flüssigen Lösungsmittelstroms, unabhängig davon, ob dieser in die Sammelzone oder in einen nachgelagerten Sammeltank eingeleitet wird.

In Schritt (v) wird so eine flüssige, (mindestens) Isocyanat und Lösungsmittel umfassende Phase und eine gasförmige, (mindestens) Chlorwasserstoff, nicht umgesetztes Phosgen, nicht verflüssigtes Isocyanat und gegebenenfalls verdampftes Lösungsmittel umfassende Phase erhalten.

Zur Gewinnung des Isocyanats aus der in Schritt (v) erhaltenen flüssigen, Isocyanat und Lösungsmittel umfassenden Phase wird diese bevorzugt weiter zu Rein-Isocyanat aufgearbeitet. Die Aufarbeitung kann mit dem gesamten in Schritt (v) anfallenden flüssigen Strom durchgeführt werden. Es ist jedoch ebenfalls möglich, nur einen Teilstrom dieser flüssigen Phase der Aufarbeitung zuzuführen und den Rest in einem **Schritt (vi)** abzukühlen und in die Quenche aus Schritt (iv) zu rezyklieren. Insbesondere betrifft daher die Erfindung auch ein Verfahren, bei welchem die in Schritt (iv) eingesetzte Quenchflüssigkeit einen Teil der in Schritt (v) erhaltenen flüssigen, Isocyanat und gegebenenfalls Lösungsmittel umfassenden Phase, welche, bevorzugt durch indirekte Kühlung (d. h. ohne direkten (stofflichen) Kontakt der der Sammelzone entnommenen Flüssigphase mit einem flüssigen Kühlmedium), abgekühlt aber ansonsten nicht in ihrer Zusammensetzung verändert wird, umfasst und insbesondere daraus besteht. Für den Zweck der indirekten Kühlung in Schritt (vi) ist insbesondere ein Wärmeaustauscher geeignet. Die Abkühlung in Schritt (vi) erfolgt bevorzugt auf eine Temperatur im Bereich von 30 °C bis 245 °C, bevorzugt 80 C bis 195 °C, besonders bevorzugt 100 °C bis 195 °C, ganz besonders bevorzugt 100 °C bis 185 °C . Da diese rezyklierte Flüssigphase außer der Abkühlung keine weitere Behandlung erfährt, also nicht weiter verändert wird, entspricht ihre Zusammensetzung in dieser Ausführungsform derjenigen der Flüssigphase in der Sammelzone.

Weiterhin kann die Effizienz der Kühlung der der Sammelzone entnommenen Flüssigphase durch einen externen Wärmeaustauscher durch das erfindungsgemäße Verfahren erheblich gesteigert werden, da die Wärmekapazität der Flüssigphase infolge ihres im Vergleich zum Stand der Technik (FIG. 1) verringerten Lösungsmittelanteils vergleichsweise hoch ist. Zudem wird bei Einsatz des erfindungsgemäßen Verfahrens die Effizienz des externen Wärmeaustauschers zusätzlich gesteigert, da das nunmehr nur vergleichsweise wenig durch Lösungsmittel verdünnte Isocyanat aufgrund der besseren Lösungseigenschaften für Rückstandskomponenten die Oberflächen des Wärmeaustauschers wirksam vor Ablagerungen schützen kann.

In einer Ausführungsform der Erfindung kann die in der Sammelzone vorliegende Flüssigphase bereits vor ihrer Entnahme durch Rückführung eines Kondensats aus der - weiter unten näher beschriebenen - Rückstandsaufarbeitung, das fast nur oder vollständig aus dem herzustellenden Isocyanat besteht (insbesondere zu 95 Massen-% bis 100 Massen-%, bezogen auf die Gesamtmasse des Kondensats), gekühlt werden. Diese Rückführung ermöglicht zum einen eine zusätzliche Kühlung der in der Sammelzone erhaltenen Flüssigphase unter Verringerung der abgedampften Isocyanat-Menge (also unter Verringerung der Schritt (v) gebildeten und der Sammelzone entnommenen Gasphase), zum anderen kann die Rückstandskonzentration im Sumpf des Reaktors durch Verdünnung niedrig gehalten werden. Der Kühlungseffekt geht einher mit der Aufheizung des Kondensats aus der Rückstandsaufarbeitung, was zu einer Energieeinsparung führt, da dieses Material ansonsten wieder in der Isocyanat-Destillationstufe aufgeheizt werden müsste.

In weiteren Ausführungsformen können ausreichend abgekühlte Isocyanat-Ströme aus beliebigen Prozess-Schritten in die Sammelzone zurückgeführt werden, um dort zu einer Abkühlung und Verdünnung zu führen.

Bevorzugt werden in Schritt (vi) 50 % bis 96 %, besonders bevorzugt 60 % bis 94 %, ganz besonders bevorzugt 70 % bis 92 % der in Schritt (v) der Sammelzone entnommenen Flüssigphase als Quenchflüssigkeit rezykliert, während der Rest einer Aufarbeitung zur Gewinnung von Rein-Isocyanat zugeführt wird. Dies geschieht entweder unmittelbar (d. h. die der Sammelzone entnommene flüssige Phase wird ohne weitere Zwischenschritte aufgearbeitet) oder nach der in der zweiten und dritten Variante vorgesehen Einspeisung eines Lösungsmittelstroms in den Sammel*tank*. In jedem Fall ermöglicht das erfindungsgemäße Verfahren in besonders bevorzugten Ausführungsformen die Bereitstellung eines der Aufarbeitung zuzuführenden flüssigen Rohprodukts (d. i. das Rohprodukt nach Zugabe des gesamten Lösungsmittelstroms aus Schritt (v)) mit einem Gehalt an organischen Lösungsmitteln von 2,0 Massen-% bis 20 Massen-%, bevorzugt von 2,0 Massen-% bis 8,0 Massen-%, besonders bevorzugt von 2,0 Massen-% bis 4,0 Massen-%, bezogen auf die Gesamtmasse des der Aufarbeitung zuzuführenden flüssigen Rohprodukts.

Die bereits erwähnte bevorzugt durchgeführte Aufarbeitung der flüssigen, Isocyanat und Lösungsmittel (aus der Zugabe in die Sammelzone und/oder in den Sammeltank) umfassenden Phase aus Schritt (v) zu Rein-Isocyanat erfolgt bevorzugt destillativ **(Schritt vii).** Insbesondere bevorzugt wird zunächst *in einem ersten **Destillationsschritt (vii-1)*** in einer sog. Lösungsmittelkolonne der Hauptteil des Lösungsmittels abgetrennt, bevor sich *in mindestens einem weiteren **Destillationsschritt (vii-2)*** eine Feinreinigung des Isocyanats anschließt. Die Aufarbeitung kann durchgeführt werden wie aus dem Stand der Technik bekannt, insbesondere wie in EP 1 371 633 A1 beschrieben. Es ist ebenfalls möglich, dem Destillationsschritt (vii-1) eine an sich aus dem Stand der Technik bekannte separate Abtrennung von gelöstem Phosgen und gelöstem Chlorwasserstoff voranzustellen **(Schritt (vii-0)).** Die Feinreinigung des Isocyanats in Schritt (viii-2) erfolgt bevorzugt in einer einzigen Destillationskolonne, die insbesondere als Trennwandkolonne ausgestaltet sein kann.

Unabhängig von der genauen Ausgestaltung der destillativen Aufarbeitung in Schritt (vii) fällt neben dem (als Destillatstrom) gewonnenen Rein-Isocyanat auch mindestens ein Destillations*sumpfstrom* an (beispielsweise der Destillationssumpfstrom der in einer bevorzugten Ausführungsform der Erfindung in Schritt (vii-2) eingesetzten Trennwandkolonne). Dieser Destillationssumpfstrom enthält den sog. Destillationsrückstand und Anteile des herzustellenden Isocyanats.

Der Destillationsrückstand enthält solche Verbindungen, die unter den für die Destillation gewählten Bedingungen von Druck und Temperatur nicht verdampfen oder die sich überhaupt nicht unzersetzt verdampfen lassen. Die schwer oder gar nicht verdampfbaren Verbindungen im Destillationsrückstand sind - sofern es sich nicht um den Phosgenierungsprozess unverändert durchlaufende Verunreinigungen aus dem eingesetzten primären Amin handelt - höhermolekulare Phosgenierungsprodukte, deren Struktur nicht immer exakt bekannt ist. So kann es sich um Verbindungen handeln, die sich (formal) von Polymerisationsprodukten des eingesetzten Amins durch Ersatz der nicht polymerisierten Amin- durch Isocyanat-Gruppen ableiten lassen. Höhermolekulare Phosgenierungsprodukte können sich zum Teil (durch Weiterreaktion) auch noch in Schritt (vii) bilden.

Der den Destillationsrückstand enthaltende Destillationssumpfstrom wird bevorzugt ebenfalls aufgearbeitet **(Schritt vii-3)),** wobei diese Aufarbeitung folgende Schritte umfasst:
a) optionale Vorkonzentrierung des Destillationssumpfstroms in einem Verdampfer durch partielle Verdampfung des in dem Destillationssumpfstrom enthaltenen herzustellenden Isocyanats, wobei ein an herzustellendem Isocyanat abgereicherter vorkonzentrierter flüssiger Strom erhalten wird;
b) Trocknung des Destillationssumpfstroms oder des in Schritt a) erhaltenen vorkonzentrierten, an herzustellendem Isocyanat abgereicherten, flüssigen Stroms in einer Trocknungsvorrichtung bei einer Temperatur im Bereich von 150 °C bis 500 °C, bevorzugt im Bereich von 185 °C bis 300 °C, besonders bevorzugt im Bereich von 200 °C bis 270 °C, und bei einem Druck im Bereich von 10 mbar_{(abs.)} bis 250 mbar_{(abs.)}, bevorzugt im Bereich von 20 mbar_{(abs.)} bis 200 mbar(_{abs}.), besonders bevorzugt im Bereich von 30 mbar_{(abs.)} bis 100 mbar_{(abs.)} durchgeführt wird, wobei herzustellendes Isocyanat unter Bildung eines festen Verfahrensprodukts verdampft und zurückgewonnen wird.

Die optionale Vorkonzentrierung durch partielle Verdampfung gemäß Schritt a) kann grundsätzlich in allen dem Fachmann bekannten Verdampfern erfolgen. Besonders bevorzugt wird Schritt a) in einem Verdampfer ausgewählt aus der Gruppe bestehend aus Dünnschichtverdampfern, Kletterverdampfern, Fallfilmverdampfern, Langrohrverdampfern, Wendelrohrverdampfern, Zwangsumlaufentspannungsverdampfer und einer Kombination dieser Apparate durchgeführt. Dabei sind Fallfilmverdampfer besonders bevorzugt. Es ist auch möglich, mehrere Verdampfer in Serie zu schalten. Bevorzugt erfolgt die Vorkonzentrierung gemäß Schritt a) bei einer Temperatur im Bereich von 120 °C bis 180 °C und bei einem Druck im Bereich von 20 mbar_{(abs.)} bis 60 mbar(_{abs}.), besonders bevorzugt bei einer Temperatur im Bereich von 130 °C bis 175 °C und bei einem Druck im Bereich von 25 mbar_{(abs.)} bis 45 mbar_{(abs.)}. Schritt a) kann kontinuierlich oder diskontinuierlich durchgeführt werden. Die kontinuierliche Verfahrensführung ist bevorzugt.

In Schritt b) erfolgt die Trocknung des in Schritt a) erhaltenen, an herzustellendem Isocyanat abgereicherten, vorkonzentrierten flüssigen Stroms bzw. - bei Verzicht auf Schritt a) - des in Destillationssumpfstromes. Für den Schritt b) geeignete Trocknungsvorrichtungen sind bevorzugt ausgewählt aus der Gruppe bestehend aus beheizten, produktumschaufelnden Vakuumtrocknern mit horizontaler Welle (bevorzugt Knetertrockner, Paddeltrockner, Schaufeltrockner; wobei jeder der genannten Trockner genau eine Welle oder mehrere Wellen, insbesondere zwei Wellen, aufweisen kann), Drehrohren, Scheibentrocknern, Bandtrocknern und Granulierschnecken. Bei der Trocknung wird das herzustellende Isocyanat verdampft und zurückgewonnen. Es verbleibt ein Feststoff, der nahezu ausschließlich aus Destillationsrückstand besteht und das herzustellende Isocyanat allenfalls noch in Spuren enthält. Der Feststoff wird bevorzugt kontinuierlich aus der Trocknungsvorrichtung ausgetragen.

Das in Schritt a) oder in den Schritten a) und b) verdampfte Isocyanat wird kondensiert. Bei Durchführung von Schritt a) und b) werden die erhaltenen Kondensate bevorzugt vereinigt. Der auf diese Weise zurückgewonnene Anteil des herzustellenden Isocyanats wird bevorzugt teilweise bis vollständig, bevorzugt vollständig, mit dem Rein-Isocyanat vereinigt oder an eine andere Stelle des Verfahrens zurückgeführt. Es ist beispielsweise möglich, den in Schritt (vii-3) gewonnenen Anteil des herzustellenden Isocyanats in Schritt (vii-2) zurückzuführen, insbesondere in den Zulauf oder in den Destillationssumpfstrom der dort eingesetzten Destillationskolonne (bei mehreren in Serie geschalteten Destillationskolonnen bevorzugt in den Zulauf oder in den Destillationssumpfstrom der letzten Destillationskolonne). In einer anderen Ausführungsform wird das erhaltene Isocyanat-Kondensat, wie bereits weiter oben beschrieben, zur direkten (Vor-)Kühlung der in der Sammelzone enthaltenden Flüssigphase verwendet.

Bevorzugt wird auch die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase aufgearbeitet **(Schritt (viii))**.

In dieser weiteren Aufarbeitung wird insbesondere nicht verflüssigtes Isocyanat zurückgewonnen und es werden Lösungsmittel (sofern in der in Schritt (v) der Sammelzone entnommenen Gasphase vorhanden), überschüssiges Phosgen und entstandenes Chlorwasserstoffgas voneinander getrennt. Das zurückgewonnene Isocyanat wird in den Prozess zurückgeführt, wie im Folgenden noch näher erläutert wird. Auch das Lösungsmittel wird aus wirtschaftlichen Gründen bevorzugt wieder dem Prozess zugeführt. Insbesondere ist es bevorzugt, das zurückgewonnene Lösungsmittel als Bestandteil, gegebenenfalls als alleinigen Bestandteil, des in Schritt (v) einzusetzen flüssigen Lösungsmittelstroms einzusetzen, wie weiter unten noch näher erläutert wird. Phosgen wird aus wirtschaftlichen Gründen ebenfalls bevorzugt wieder der Reaktion zugeführt, und zwar insbesondere dem Schritt (i). Der zurückgewonnene Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor, das wieder in den Isocyanatprozess zurückgeführt werden kann, liefert. Zu diesen Recyclingverfahren zählen die katalytische Oxidation von Chlorwasserstoff, z. B. nach dem Deacon-Verfahren, die Elektrolyse von gasförmigem Chlorwasserstoff sowie die Elektrolyse einer wässrigen Lösung von Chlorwasserstoff (Salzsäure), erhalten durch Absorption in Wasser des gebildeten Chlorwasserstoffs.

In Schritt (viii) wird zunächst nicht verflüssigtes Isocyanat aus der in Schritt (v) der Sammelzone entnommenen gasförmigen Phase zurückgewonnen.

Zur Durchführung des Schrittes (viii) kann so verfahren werden, dass man zunächst die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase in einem **Schritt (viii-1)** durch Abkühlung (d. h. indirekte Abkühlung [also ohne stofflichen Kontakt mit einem flüssigen Wärmeträgermedium]; diese Abkühlung wird bevorzugt in einem *Wärmeaustauscher* durchgeführt.) unter Erhalt eines flüssigen, gegebenenfalls Lösungsmittel und Isocyanat umfassenden Stroms und eines gasförmigen, Chlorwasserstoff, Phosgen, gegebenenfalls nicht verflüssigtes Isocyanat und gegebenenfalls nicht verflüssigtes Lösungsmittel umfassenden Stroms kondensiert, in einer besonderen Ausgestaltung dieser Ausführungsform nur teilweise (d. h. die Verflüssigung von Lösungsmittel (sofern vorhanden) und Isocyanat erfolgt bewusst nicht vollständig) kondensiert.

Bei ausreichender Kondensation des Isocyanats in der Kondensation (viii-1) kann die erhaltene gasförmige Phase - da weitgehend bis vollständig von Isocyanat befreit - direkt der weiteren Aufarbeitung zur Zurückgewinnung von Phosgen und Chlorwasserstoff zugeführt werden. Die kondensierte Phase kann, je nach Zusammensetzung, entweder der Aufarbeitung in Schritt (vii) zugeführt oder - bevorzugt - in Schritt (v) als flüssiger Lösungsmittelstrom, der der Sammelzone oder einem strömungstechnisch nachgelagerten Sammeltank zugeführt wird (oder als Teil desselben), eingesetzt werden. Letzteres setzt natürlich voraus, dass die kondensierte Phase ausreichend Lösungsmittel enthält, was in der ersten und dritten Variante der Erfindung regelmäßig der Fall ist (da dort ein Teil des in die Sammelzone eingetragenen Lösungsmittels verdampft und im Kondensator aus (viii-1) wieder verflüssigt wird) und in der zweiten Variante dann der Fall ist, wenn die Quenchflüssigkeit aus Schritt (iv) genügend Lösungsmittel umfasst.

Bei nur teilweiser Kondensation in Schritt (viii-1) ist es bevorzugt, den erhaltenen gasförmigen, Chlorwasserstoff, Phosgen, gegebenenfalls nicht verflüssigtes Isocyanat und gegebenenfalls nicht verflüssigtes Lösungsmittel umfassenden Strom zur Abtrennung von restlichem Isocyanat in einem **Schritt (viii-2)** in eine Auswaschkolonne zu leiten und dort mit mindestens einem flüssigen Lösungsmittelstrom (d. h. ein Strom, der mehr als 50,0 Massen-%, insbesondere mindestens 51,0 Massen-%, bevorzugt mindestens 80,0 Massen-%, besonders bevorzugt mindestens 90,0 Massen-%, ganz besonders bevorzugt mindestens 99,0 Massen-%, bezogen auf die Gesamtmasse des Lösungsmittelstroms, an organischen Lösungsmitteln enthält) als Auswaschflüssigkeit in Kontakt zu bringen, wobei ein flüssiger, Lösungsmittel und Isocyanat umfassender Strom und ein gasförmiger, Chlorwasserstoff und Phosgen umfassender Strom erhalten werden.

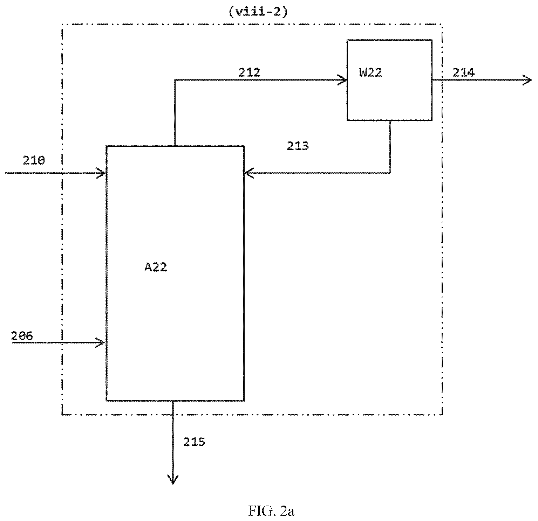

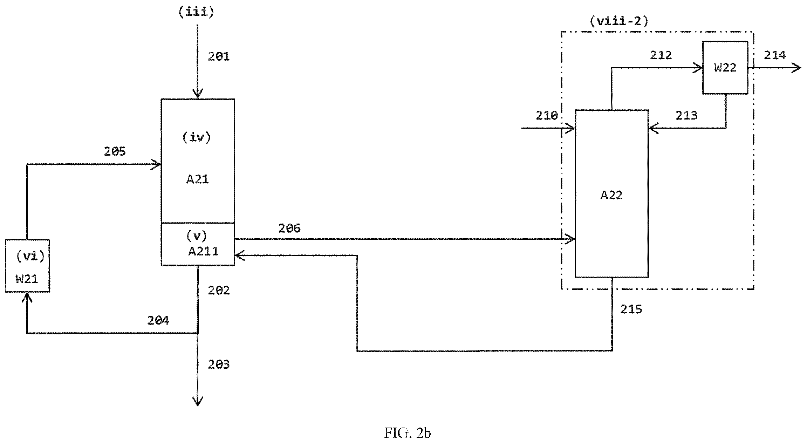

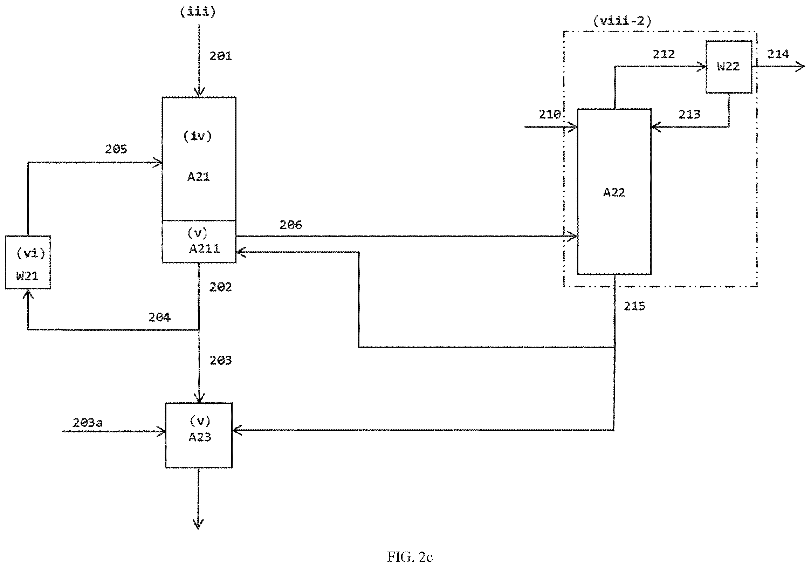

Geeignete Auswaschkolonnen sind dem Fachmann bekannt. Beispielhaft seinen Packungskolonnen und Bodenkolonnen genannt. Im Folgenden wird der Betrieb einer solchen Auswaschkolonne in Schritt (viii-2) anhand von **FIG. 2a** allgemein erläutert (FIG. 2a ist eine Ausschnittvergrößerung der Auswaschkolonne aus FIG. 2b und FIG. 2c; *die folgende Beschreibung ist jedoch nicht auf die Ausführungsformen gemäß* *FIG. 2b* *und* *FIG. 2c* *beschränkt, sondern gilt für alle Ausführungsformen der Erfindung):*

Der von Isocyanat durch Auswaschung zu befreiende gasförmige Stoffstrom (206 in FIG. 2a) wird der Auswaschkolonne (A22) zugeleitet. Die dazu erforderliche Auswaschflüssigkeit wird am Kopf der Auswaschkolonne (A22) aufgegeben. Die Brüden (212) der Auswaschkolonne durchlaufen einen Kondensator (*Brüdenkondensator* W22). Selbstverständlich können auch mehrere solcher Kondensatoren, insbesondere zwei, in Reihe geschaltet, eingesetzt werden; allgemein gilt, dass wenn bei der Beschreibung der Erfindung von "einem" Apparat gesprochen wird und/oder in den Zeichnungen nur ein Apparat gezeigt ist, dies, sofern nicht anders vermerkt, Ausführungsformen, in denen zwei oder mehr Apparate für den jeweiligen Zweck eingesetzt werden, nicht ausschließt. Im Kondensator (W22) verflüssigte Anteile (213) werden auf den Kopf der Auswaschkolonne zurückgegeben und so in dieselbe rezykliert. Die nicht kondensierten Anteile (214), die überwiegend Chlorwasserstoffgas und Phosgen enthalten, werden, wie weiter unten beschrieben, bevorzugt zur Rückgewinnung von Chlorwaserstoff und Phosgen aufgearbeitet. Je größer der Gehalt an Isocyanat im zugeführten Stoffstrom (206) ist, desto größer muss der am Kopf der Auswaschkolonne zugeführte Strom an Auswaschflüssigkeit gewählt werden, und desto mehr Trennstufen werden für eine zuverlässige Rückhaltung benötigt. Die insgesamt in Schritt (viii-2) eingesetzte Auswaschflüssigkeit setzt sich aus dem Kondensat (213) des Kondensators (W22) und zusätzlich zugeführter Waschflüssigkeit (210) zusammen. Diese zusätzlich zugeführte Waschflüssigkeit (210) ist ein Lösungsmittelstrom, der ein organisches Lösungsmittel ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel enthält.

Es ist möglich, die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase unmittelbar, also ohne Durchlaufen der Kondensation in Schritt (viii-1), in eine Auswaschkolonne aus Schritt (viii-2) zu leiten und dort mit mindestens einem flüssigen Lösungsmittelstrom als Auswaschflüssigkeit in Kontakt zu bringen, wobei ein flüssiger, Lösungsmittel und Isocyanat umfassender Strom und ein gasförmiger, Chlorwasserstoff und Phosgen umfassender Strom erhalten werden. Als Auswaschflüssigkeit wird bevorzugt ein flüssiger Lösungsmittelstrom enthaltend ein Lösungsmittel ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel gewählt. Bevorzugt wird das gleiche Lösungsmittel verwendet, das in dem in die Sammelzone aus Schritt (v) oder den nachgelagerten Sammeltank geleiteten flüssigen Lösungsmittelstrom enthalten ist. Sofern die in Schritt (iv) eingesetzte Quenchflüssigkeit ebenfalls Lösungsmittel enthält, ist dieses bevorzugt ebenfalls ausgewählt aus der zuvor genannten Gruppe.

In der Mehrzahl der zuvor geschilderten Ausführungsformen des Schritts (viii) fallen flüssige Lösungsmittelströme an, die geringe Mengen an Isocyanat enthalten (in dem in Schritt (viii-1) eingesetzten Kondensator und in der in Schritt (viii-2) eingesetzten Auswaschkolonne) und sich dazu eignen, in Schritt (v) als flüssiger Lösungsmittelstrom in die Sammelzone oder einen nachgelagerten Sammeltank geleitet zu werden. Insbesondere betrifft daher die Erfindung auch ein Verfahren, bei welchem der in Schritt (v) in die Sammelzone oder einen nachgelagerten Sammeltank geleitete flüssige Lösungsmittelstrom den in Schritt (viii-1) erhaltenen flüssigen, Lösungsmittel und Isocyanat umfassenden Strom und/oder den in Schritt (viii-2), insofern durchgeführt, erhaltenen flüssigen, Lösungsmittel und Isocyanat umfassenden Strom umfasst und insbesondere keine weiteren Ströme umfasst. Dies ist exemplarisch für die Ausführungsform unter Einsatz der Auswaschkolonne aus Schritt (viii-2) (ohne vorige Kondensation in Schritt (viii-1)) in **FIG. 2b** gezeigt. Im Unterschied zu FIG. 1 beginnen alle Bezugszeichen mit der Ziffer 2 und haben ansonsten die gleiche Bedeutung wie dort. Der Lösungsmittelgehalt des Stroms 215 wird eingestellt durch die Wahl der Zusammensetzung und des Mengenstroms der zugeführten Auswaschflüssigkeit (210) und durch die Wahl der Kondensationsleistung des Kondensators W22, welche für die Zusammensetzung und den Mengenstrom des Rückführstromes an rezykliertem Brüdenkondensat (213) wesentlich ist. Als Auswaschflüssigkeit (210) für die Auswaschkolonne A22 aus Schritt (viii-2) eignet sich ein Lösungsmittelstrom wie zuvor bei der allgemeinen Beschreibung der Auswaschkolonne definiert. Dabei ist selbstverständlich zu beachten, dass die Bedingungen - also insbesondere die Mengenströme an zu waschender Gasphase aus der Sammelzone (Schritt (v); 206), an zusätzlich zugeführter Auswaschflüssigkeit (210) und an zurückgeführtem Brüdenkondensat (213) -, so gewählt werden müssen, dass die erfindungsgemäßen Anforderungen im Hinblick auf den maximalen Lösungsmittelgehalt der Quenchflüssigkeit in Schritt (iv) - hier Strom 205 - eingehalten werden.

**FIG. 2c** zeigt eine apparative Ausgestaltung der Erfindung, die insbesondere in der dritten Variante (aber auch in der ersten und zweiten Variante) eingesetzt werden kann:
Der nicht rezyklierte Anteil (203) der flüssigen Phase (202) aus der Sammelzone (A211) wird in den Sammeltank (A23) geleitet. Dieser Sammeltank (A23) kann zusätzlich mit entsprechenden flüssigen Strömen (203a) aus den Sammelzonen *von anderen Reaktionsstraßen* gespeist werden. Der flüssige Lösungsmittelstrom (215) aus der Auswaschkolonne (A22) kann in den Sammeltank (A211) und in die Sammelzone (A23) geleitet werden. Die jeweiligen Leitungen sind dabei mit Regelventilen versehen (in der Abbildung nicht gezeigt), die es ermöglichen, den in die Sammelzone (A211) und den Sammeltank (A23) geleiteten Anteil des Lösungsmittelstroms (215) stufenlos zu regulieren. Dabei kann auch einer der beiden Ströme völlig unterbrochen werden, entsprechend der ersten Variante der Erfindung, wenn der Lösungsmittelstrom (215) in den Sammeltank (A23) unterbrochen wird bzw. entsprechend der zweiten Variante der Erfindung, wenn der Lösungsmittelstrom (215) in die Sammelzone (A211) unterbrochen wird. Es ist mit diesem apparativen Aufbau möglich, innerhalb eines Produktionszyklus zwischen den drei Varianten der Erfindung zu wechseln, was die Flexibilität des Verfahrens steigert.

Jede Reaktionsstraße kann dabei ihre eigene Auswaschkolonne (A22) haben, in welchem Fall die dort anfallenden Ströme (215a) ebenfalls in den allen Reaktionsstraßen gemeinsamen Sammeltank (A23) geleitet werden. Es ist jedoch ebenfalls möglich, dass verschiedene Reaktionsstraßen eine gemeinsame Auswaschkolonne (A22) haben, wobei dann die in den jeweiligen Sammelzonen erhaltenen gasförmigen Phasen (206a) ebenfalls in die gemeinsame Auswaschkolonne (A22) geleitet werden. Diese beiden Alternativen sind in der Abbildung durch gestrichelte Pfeile dargestellt.

Der in Schritt (viii-1) oder Schritt (viii-2) erhaltene gasförmige Strom (214 in FIG. 2a-c) wird bevorzugt in einem **Schritt (viii-3)** in an sich bekannter Weise von überschüssigem Phosgen befreit. Dies kann mittels einer Kühlfalle, Absorption in einem inerten Lösungsmittel (bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel) oder durch Adsorption und Hydrolyse an Aktivkohle erfolgen. Bevorzugt erfolgt dieser Schritt wie in WO2011/003532 A1 beschrieben; siehe insbesondere S. 11, Z. 31 bis S. 25, Z. 15. Der in der Phosgenabtrennung erhaltene, im Wesentlichen aus Chlorwasserstoff bestehende Gasstrom kann in der in der zuvor geschilderten Weise weiter verarbeitet werden.

In allen Schritten des erfindungsgemäßen Verfahrens, in welchen ein organisches Lösungsmittel eingesetzt wird, wird bevorzugt das gleiche organische Lösungsmittel eingesetzt, das insbesondere ausgewählt wird aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel. Besonders bevorzugt ist der Einsatz von ortho-Dichlorbenzol.

Das erfindungsgemäße Verfahren weist eine Reihe von Vorteilen auf, insbesondere:
a) Verringerung der internen Lösungsmittelkreisläufe;
b) Verkleinerung der Lösungsmitteldestillation gemäß Schritt (vii-1)
c) entsprechende Einsparung von einzubringender Verdampfungs-Energie;
d) Reduktion der die Sammelzone verlassenden Brüdenvolumina und dadurch Verkleinerung nachgeschalteter Apparate (z. B. der Isocyanat-Auswaschkolonne);
e) höhere Effizienz des Reaktorsumpfkühlers durch maximierten Wärmeübergang.

## Patentansprüche

1. Verfahren zur Herstellung eines Isocyanats durch Phosgenierung des korrespondierenden primären Amins in der Gasphase umfassend die Schritte:
(i) Bereitstellung eines gasförmigen Stroms eines primären Amins;
(ii) Bereitstellung eines gasförmigen Phosgenstroms;
(iii) Vermischen des gasförmigen Stroms des primären Amins aus Schritt (i) und des gasförmigen Phosgenstroms aus Schritt (ii) zu einem gasförmigen Reaktionsgemisch unter Einhaltung eines auf primäre Aminogruppen bezogenen stöchiometrischen Überschusses an Phosgen in einer Mischzone und Führen des so erhaltenen gasförmigen Reaktionsgemisches durch eine Reaktionszone zur Umsetzung des primären Amins mit Phosgen unter Erhalt eines gasförmigen Reaktionsproduktgemisches;
(iv) Abkühlen des nach Durchlaufen der Reaktionszone aus Schritt (iii) erhaltenen gasförmigen Reaktionsproduktgemisches durch Inkontaktbringen mit mindestens einem Strom einer Quenchflüssigkeit in einer Quenchzone, wobei die insgesamt eingesetzte Quenchflüssigkeit, bezogen auf ihre Gesamtmasse, organische Lösungsmittel in einem Anteil von maximal 50,0 Massen-% umfasst, wobei der Rest zu 100 Massen-% aus mindestens dem herzustellenden Isocyanat besteht, unter Erhalt eines Gemisches aus Reaktionsproduktgemisch und Quenchflüssigkeit;
(v) Führen des in Schritt (iv) erhaltenen Gemisches aus Reaktionsproduktgemisch und Quenchflüssigkeit in eine Sammelzone zur Trennung von flüssiger und gasförmiger Phase unter Erhalt einer flüssigen, Isocyanat und gegebenenfalls Lösungsmittel umfassenden Phase und einer gasförmigen, Chlorwasserstoff, nicht umgesetztes Phosgen, nicht verflüssigtes Isocyanat und gegebenenfalls verdampftes Lösungsmittel umfassenden Phase, wobei ein flüssiger Lösungsmittelstrom umfassend mehr als 50,0 Massen-% an organischen Lösungsmitteln, bezogen auf die Gesamtmasse des flüssigen Lösungsmittelstroms, in die Sammelzone und/oder in einen strömungstechnisch nach der Sammelzone angeordneten Sammeltank für die in der Sammelzone erhaltene flüssige Phase eingeleitet wird.

2. Verfahren gemäß Anspruch 1, bei welchem der flüssige Lösungsmittelstrom in Schritt (v) nur in die Sammelzone geleitet wird, wobei der flüssige Lösungsmittelstrom in einem solchen Mengenstrom in die Sammelzone geleitet wird, dass sein Anteil an der Gesamtmasse der in der Sammelzone erhaltenen flüssigen, Isocyanat und Lösungsmittel umfassenden Phase im Bereich von 20 Massen-% bis 80 Massen-% liegt.

3. Verfahren gemäß Anspruch 1, bei welchem der flüssige Lösungsmittelstrom in Schritt (v) (a) in den Sammeltank oder (b) in den Sammeltank und in die Sammelzone geleitet wird, wobei der Mengenstrom des flüssigen Lösungsmittelstroms jeweils so gewählt wird, dass sein Anteil an der Gesamtmasse der im Sammeltank erhaltenen flüssigen, Isocyanat und Lösungsmittel umfassenden Phase im Bereich von 20 Massen-% bis 80 Massen-% liegt.

4. Verfahren gemäß einem der vorstehenden Ansprüche, bei welchem die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase in einem Schritt (viii-1) durch indirekte Abkühlung unter Erhalt eines flüssigen, Isocyanat und gegebenenfalls Lösungsmittel umfassenden Stroms und eines gasförmigen, Chlorwasserstoff, Phosgen, gegebenenfalls nicht verflüssigtes Isocyanat und gegebenenfalls nicht verflüssigtes Lösungsmittel umfassenden Stroms kondensiert wird, insbesondere teilweise kondensiert wird.

5. Verfahren gemäß Anspruch 4, bei welchem der in Schritt (viii-1) erhaltene gasförmige, Chlorwasserstoff, Phosgen, gegebenenfalls nicht verflüssigtes Isocyanat und gegebenenfalls nicht verflüssigtes Lösungsmittel umfassende Strom in einem Schritt (viii-2) in eine Auswaschkolonne geleitet und dort mit mindestens einem flüssigen Lösungsmittelstrom als Auswaschflüssigkeit in Kontakt gebracht wird, wobei ein flüssiger, Lösungsmittel und Isocyanat umfassender Strom und ein gasförmiger, Chlorwasserstoff und Phosgen umfassender Strom erhalten werden.

6. Verfahren gemäß einem der Ansprüche 1 bis 3, bei welchem die in Schritt (v) erhaltene gasförmige, Chlorwasserstoff, nicht umgesetztes Phosgen, nicht verflüssigtes Isocyanat und gegebenenfalls verdampftes Lösungsmittel umfassende Phase in einem Schritt (viii-2) in eine Auswaschkolonne geleitet und dort mit mindestens einem flüssigen Lösungsmittelstrom als Auswaschflüssigkeit in Kontakt gebracht wird, wobei ein flüssiger, Lösungsmittel und Isocyanat umfassender Strom und ein gasförmiger, Chlorwasserstoff und Phosgen umfassender Strom erhalten werden.

7. Verfahren gemäß einem der Ansprüche 4 oder 5, bei welchem die in der Sammelzone erhaltene gasförmige, Chlorwasserstoff, nicht umgesetztes Phosgen und nicht verflüssigtes Isocyanat umfassende Phase verdampftes Lösungsmittel umfasst und der in Schritt (v) in die Sammelzone oder den Sammeltank geleitete flüssige Lösungsmittelstrom den in Schritt (viii-1) erhaltenen flüssigen, Isocyanat und Lösungsmittel umfassenden Strom umfasst.

8. Verfahren gemäß einem der Ansprüche 5 bis 7, bei welchem Schritt (viii-2) durchgeführt wird und der in Schritt (v) in die Sammelzone oder den Sammeltank geleitete flüssige Lösungsmittelstrom den in Schritt (viii-2) erhaltenen flüssigen, Lösungsmittel und Isocyanat umfassenden Strom umfasst.

9. Verfahren gemäß einem der Ansprüche 7 oder 8, bei welchem der Lösungsmittelstrom aus Schritt (v) keine weiteren Bestandteile außer den flüssigen Isocyanat und Lösungsmittel umfassenden Strömen aus Schritt (viii-1) und/oder Schritt (viii-2) umfasst.

10. Verfahren gemäß einem der vorstehenden Ansprüche, bei welchem in Schritt (v) der flüssige Lösungsmittelstrom zumindest zeitweise in die Sammelzone und in den Sammeltank geleitet wird, wobei die in die Sammelzone und den Sammeltank führenden Leitungen für den Lösungsmittelstrom unabhängig voneinander regelbar sind.

11. Verfahren gemäß einem der vorstehenden Ansprüche, bei welchem ein Teil der in Schritt (v) in der Sammelzone erhaltenen flüssigen, Isocyanat und gegebenenfalls Lösungsmittel umfassenden Phase in einem Schritt (vi) abgekühlt aber ansonsten nicht in seiner Zusammensetzung verändert und in die Quenchzone aus Schritt (iv) rezykliert wird.

12. Verfahren gemäß einem der vorstehenden Ansprüche, bei welchem mindestens ein Teil der in Schritt (v) in der Sammelzone erhaltenen flüssigen, Isocyanat und gegebenenfalls Lösungsmittel umfassenden Phase in einem Schritt (vii) zu Rein-Isocyanat aufgearbeitet wird.

13. Verfahren gemäß einem der vorstehenden Ansprüche, bei welchem der in Schritt (v) in die Sammelzone oder den Sammeltank geleitete flüssige Lösungsmittelstrom ausschließlich Lösungsmittel ausgewählt aus der Gruppe bestehend aus aliphatischen Kohlenwasserstoffen, aromatischen Kohlenwasserstoffen ohne Halogensubstitution, aromatischen Kohlenwasserstoffen mit Halogensubstitution und Mischungen der vorgenannten Lösungsmittel enthält, und bei welchem
• das in der in Schritt (iv) eingesetzten Quenchflüssigkeit optional vorhandene Lösungsmittel, sofern vorhanden, und
• das in dem in Schritt (viii-2), sofern durchgeführt, als Auswaschflüssigkeit eingesetzten flüssigen Lösungsmittelstrom enthaltende Lösungsmittel
jeweils das gleiche ist wie das in dem in Schritt (v) in die Sammelzone oder den Sammeltank geleiteten flüssigen Lösungsmittelstrom enthaltende Lösungsmittel.

14. Verfahren gemäß einem der vorstehenden Ansprüche, bei welchem das primäre Amin ausgewählt ist aus der Gruppe bestehend aus Toluylendiamin, Diphenylmethandiamin, Xylylendiamin, 1,5-Pentandiamin, 1,6-Hexamethylendiamin, Isophorondiamin, Diaminodicyclohexylmethan und Mischungen der vorgenannten Verbindungen.

15. Verfahren gemäß einem der vorstehenden Ansprüche, bei welchem in allen Schritten des Verfahrens, in denen ein organisches Lösungsmittel eingesetzt wird, das gleiche organische Lösungsmittel eingesetzt wird, und dieses organische Lösungsmittel ausgewählt ist aus der Gruppe bestehend aus aliphatischen Kohlenwasserstoffen, aromatischen Kohlenwasserstoffen ohne Halogensubstitution, aromatischen Kohlenwasserstoffen mit Halogensubstitution und Mischungen der vorgenannten organischen Lösungsmittel.

## Claims

1. Process for preparing an isocyanate by phosgenating the corresponding primary amine in the gas phase, comprising the steps of:
(i) providing a gaseous stream of a primary amine;
(ii) providing a gaseous phosgene stream;
(iii) mixing the gaseous stream of the primary amine from step (i) and the gaseous phosgene stream from step (ii) to give a gaseous reaction mixture while maintaining a stoichiometric excess of phosgene relative to primary amino groups in a mixing zone and conducting the gaseous reaction mixture thus obtained through a reaction zone for reaction of the primary amine with phosgene to obtain a gaseous reaction product mixture;
(iv) cooling the gaseous reaction product mixture obtained after passage through the reaction zone from step (iii) by contacting with at least one stream of a quench liquid in a quench zone, where the total quench liquid used, based on its total mass, comprises organic solvents in a proportion of at most 50.0% by mass, where the remainder to 100% by mass consists of at least the isocyanate to be prepared, to obtain a mixture of reaction product mixture and quench liquid;
(v) conducting the mixture of reaction product mixture and quench liquid obtained in step (iv) into a collecting zone for separation of liquid phase and gaseous phase to obtain a liquid phase comprising isocyanate and optionally solvent and a gaseous phase comprising hydrogen chloride, unconverted phosgene, unliquefied isocyanate and any evaporated solvent, with introduction of a liquid solvent stream comprising more than 50.0% by mass of organic solvents, based on the total mass of the liquid solvent stream, into the collecting zone and/or into a collecting tank for the liquid phase obtained in the collecting zone which is disposed downstream in flow direction of the collecting zone.

2. Process according to Claim 1, in which the liquid solvent stream in step (v) is guided solely into the collecting zone, where the liquid solvent stream is guided into the collecting zone at such a flow rate that its proportion in the total mass of the liquid phase comprising isocyanate and solvent which is obtained in the collecting zone is in the range from 20% by mass to 80% by mass.

3. Process according to Claim 1, in which the liquid solvent stream in step (v) is guided (a) into the collecting tank or (b) into the collecting tank and into the collecting zone, where the flow rate of the liquid solvent stream in each case is chosen such that its proportion in the total mass of the liquid phase comprising isocyanate and solvent which is obtained in the collecting tank is in the range from 20% by mass to 80% by mass.

4. Process according to any of the preceding claims, in which the gaseous phase comprising hydrogen chloride, unconverted phosgene, unliquefied isocyanate and any evaporated solvent which is obtained in step (v) is condensed, especially is partly condensed, in a step (viii-1) by indirect cooling to obtain a liquid stream comprising isocyanate and optionally solvent and a gaseous stream comprising hydrogen chloride, phosgene, any unliquefied isocyanate and any unliquefied solvent.

5. Process according to Claim 4, in which the gaseous stream comprising hydrogen chloride, phosgene, any unliquefied isocyanate and any unliquefied solvent which is obtained in step (viii-1) is guided, in a step (viii-2), into a scrubbing column, where it is contacted with at least one liquid solvent stream as scrubbing liquid, giving a liquid stream comprising solvent and isocyanate and a gaseous stream comprising hydrogen chloride and phosgene.

6. Process according to any of Claims 1 to 3, in which the gaseous phase comprising hydrogen chloride, unconverted phosgene, unliquefied isocyanate and any evaporated solvent which is obtained in step (v) is guided, in a step (viii-2), into a scrubbing column, where it is contacted with at least one liquid solvent stream as scrubbing liquid, giving a liquid stream comprising solvent and isocyanate and a gaseous stream comprising hydrogen chloride and phosgene.

7. Process according to either of Claims 4 and 5, in which the gaseous phase comprising hydrogen chloride, unconverted phosgene and unliquefied isocyanate which is obtained in the collecting zone comprises evaporated solvent and the liquid solvent stream guided into the collecting zone or collecting tank in step (v) comprises the liquid stream comprising isocyanate and solvent which is obtained in step (viii-1).

8. Process according to any of Claims 5 to 7, in which step (viii-2) is conducted and the liquid solvent stream guided into the collecting zone or collecting tank in step (v) comprises the liquid stream comprising solvent and isocyanate which is obtained in step (viii-2).

9. Process according to either of Claims 7 and 8, in which the solvent stream from step (v) does not comprise any further constituents apart from the liquid streams comprising isocyanate and solvent from step (viii-1) and/or step (viii-2).

10. Process according to any of the preceding claims, in which, in step (v), the liquid solvent stream is guided at least temporarily into the collecting zone and into the collecting tank, where the conduits for the solvent stream that lead into the collecting zone and the collecting tank are controllable independently of one another.

11. Process according to any of the preceding claims, in which a portion of the liquid phase comprising isocyanate and optionally solvent which is obtained in the collecting zone in step (v) is cooled down in a step (vi) but is otherwise unchanged in its composition and is recycled into the quench zone from step (iv).

12. Process according to any of the preceding claims, in which at least a portion of the liquid phase comprising isocyanate and optionally solvent which is obtained in the collecting zone in step (v) is worked up in a step (vii) to give pure isocyanate.

13. Process according to any of the preceding claims, in which the liquid solvent stream guided into the collecting zone or collecting tank in step (v) contains exclusively solvents selected from the group consisting of aliphatic hydrocarbons, aromatic hydrocarbons having no halogen substitution, aromatic hydrocarbons having halogen substitution and mixtures of the aforementioned solvents, and in which
• the solvent optionally present in the quench liquid used in step (iv), if present, and
• the solvent contained in the liquid solvent stream used as scrubbing liquid in step (viii-2), if conducted,
is the same in each case as the solvent contained in the liquid solvent stream guided into the collecting zone or collecting tank in step (v).

14. Process according to any of the preceding claims, in which the primary amine is selected from the group consisting of tolylenediamine, diphenylmethanediamine, xylylenediamine, pentane-1,5-diamine, hexamethylene-1,6-diamine, isophoronediamine, diaminodicyclohexylmethane and mixtures of the aforementioned compounds.

15. Process according to any of the preceding claims, in which the same organic solvent is used in all steps of the process in which an organic solvent is used, and this organic solvent is selected from the group consisting of aliphatic hydrocarbons, aromatic hydrocarbons having no halogen substitution, aromatic hydrocarbons having halogen substitution and mixtures of the aforementioned organic solvents.

## Revendications

1. Procédé de préparation d'un isocyanate par phosgénation de l'amine primaire correspondante en phase gazeuse, comprenant les étapes suivantes :
(i) fourniture d'un courant gazeux d'une amine primaire ;
(ii) fourniture d'un courant de phosgène gazeux ;
(iii) mélange du courant gazeux de l'amine primaire de l'étape (i) et du courant de phosgène gazeux de l'étape (ii) pour obtenir un mélange réactionnel gazeux avec maintien d'un excès stœchiométrique, par rapport aux groupes amino primaires, de phosgène dans une zone de mélange, et envoi du mélange réactionnel gazeux ainsi obtenu dans une zone de réaction pour réaction de l'amine primaire avec le phosgène, pour obtenir un mélange de produits de réaction gazeux ;
(iv) refroidissement du mélange de produits de réaction gazeux obtenu après passage dans la zone de réaction de l'étape (iii), par mise en contact d'au moins un courant d'un liquide de refroidissement dans une zone de refroidissement, le liquide de refroidissement utilisé en totalité comprenant, par rapport à sa masse totale, des solvants organiques selon une proportion au maximum de 50,0 % en masse, le complément à 100 % en masse étant constitué d'au moins l'isocyanate à préparer, avec obtention d'un mélange du mélange de produits de réaction et du liquide de refroidissement ;
(v) envoi du mélange obtenu dans l'étape (iv) du mélange de produits de réaction et du liquide de refroidissement dans une zone d'accumulation pour séparer la phase liquide et la phase gazeuse, avec obtention d'une phase liquide, comprenant l'isocyanate et éventuellement des solvants, et d'une phase gazeuse, comprenant du chlorure d'hydrogène, du phosgène n'ayant pas réagi, l'isocyanate non liquéfié et éventuellement un solvant évaporé, un courant de solvant liquide, comprenant plus de 50,0 % en masse de solvants organiques, par rapport à la masse totale du courant de solvant liquide, étant introduit dans la zone d'accumulation et/ou dans un réservoir disposé fluidiquement en aval de la zone d'accumulation, destiné à la phase liquide obtenue dans la zone d'accumulation.

2. Procédé selon la revendication 1, dans lequel le courant de solvant liquide de l'étape (v) est envoyé uniquement dans la zone d'accumulation, le courant de solvant liquide étant envoyé dans la zone d'accumulation avec un débit massique tel que sa proportion, par rapport à la masse totale de la phase liquide obtenue dans la zone d'accumulation, comprenant l'isocyanate et le solvant, soit comprise dans la plage de 20 % en masse à 80 % en masse.

3. Procédé selon la revendication 1, dans lequel le courant de solvant liquide de l'étape (v) est envoyé (a) dans le réservoir ou (b) dans le réservoir et dans la zone d'accumulation, le débit massique du courant de solvant liquide étant dans chaque cas choisi de telle sorte que sa proportion par rapport à la masse totale de la phase liquide obtenue dans le réservoir, comprenant l'isocyanate et le solvant, soit comprise dans la plage de 20 % en masse à 80 % en masse.

4. Procédé selon l'une des revendications précédentes, dans lequel la phase gazeuse obtenue dans l'étape (v), comprenant du chlorure d'hydrogène, du phosgène n'ayant pas réagi, l'isocyanate non liquéfié et éventuellement un solvant évaporé, est condensée, en particulier partiellement condensée, dans une étape (viii-1) par refroidissement indirect avec obtention d'un courant liquide, comprenant l'isocyanate et éventuellement le solvant, et d'un courant gazeux, comprenant du chlorure d'hydrogène, du phosgène, éventuellement l'isocyanate non liquéfié et éventuellement le solvant non liquéfié.

5. Procédé selon la revendication 4, dans lequel la phase gazeuse obtenue dans l'étape (viii-1), comprenant le chlorure d'hydrogène, le phosgène, éventuellement l'isocyanate non liquéfié et éventuellement le solvant non liquéfié, est envoyée dans une étape (viii-2) dans une colonne d'extraction par lavage, et y est mise en contact avec au moins un courant de solvant liquide servant de liquide d'extraction par lavage, avec obtention d'un courant liquide comprenant le solvant et l'isocyanate et d'un courant gazeux comprenant du chlorure d'hydrogène et du phosgène.

6. Procédé selon l'une des revendications 1 à 3, dans lequel la phase gazeuse obtenue dans l'étape (v), comprenant le chlorure d'hydrogène, le phosgène n'ayant pas réagi, l'isocyanate non liquéfié et éventuellement le solvant évaporé, est envoyée dans une étape (viii-2) dans une colonne d'extraction par lavage et y est mise en contact avec au moins un courant de solvant liquide servant de liquide d'extraction par lavage, avec obtention d'un courant liquide comprenant le solvant et l'isocyanate et d'un courant gazeux comprenant du chlorure d'hydrogène et du phosgène.

7. Procédé selon l'une des revendications 4 ou 5, dans lequel la phase gazeuse obtenue dans la zone d'accumulation, comprenant le chlorure d'hydrogène, le phosgène n'ayant pas réagi et l'isocyanate non liquéfié, comprend un solvant évaporé, et le courant de solvant liquide envoyé dans l'étape (v) dans la zone d'accumulation ou dans le réservoir comprend le courant liquide obtenu dans l'étape (viii-1), comprenant l'isocyanate et le solvant.

8. Procédé selon l'une des revendications 5 à 7, dans lequel l'étape (viii-2) est mise en œuvre, et le courant de solvant liquide envoyé dans l'étape (v) dans la zone d'accumulation ou dans le réservoir comprend le courant liquide obtenu dans l'étape (viii-2), comprenant le solvant et l'isocyanate.

9. Procédé selon l'une des revendications 7 ou 8, dans lequel le courant de solvant de l'étape (v) ne comprend aucun autre constituant à l'exception des courants liquides comprenant l'isocyanate et le solvant, de l'étape (viii-1) et/ou de l'étape (viii-2).

10. Procédé selon l'une des revendications précédentes, dans lequel dans l'étape (v), le courant de solvant liquide est au moins en partie envoyé dans la zone d'accumulation et dans le réservoir, les conduites allant dans la zone d'accumulation et dans le réservoir, destinées au courant de solvant, pouvant être réglées indépendamment l'une de l'autre.

11. Procédé selon l'une des revendications précédentes, dans lequel une partie de la phase liquide obtenue dans l'étape (v) dans la zone d'accumulation, comprenant l'isocyanate et éventuellement le solvant, est refroidie dans une étape (vi), mais par ailleurs sans modification de sa composition, et est recyclée dans la zone de refroidissement de l'étape (iv).

12. Procédé selon l'une des revendications précédentes, dans lequel au moins une partie de la phase liquide obtenue dans l'étape (v) dans la zone d'accumulation, comprenant l'isocyanate et éventuellement le solvant, est retraitée dans une étape (vii) pour obtenir de l'isocyanate pur.

13. Procédé selon l'une des revendications précédentes, dans lequel le courant de solvant liquide envoyé dans l'étape (v) dans la zone d'accumulation ou dans le réservoir contient exclusivement des solvants choisis dans le groupe consistant en les hydrocarbures aliphatiques, les hydrocarbures aromatiques sans substitution par un halogène, les hydrocarbures aromatiques avec une substitution par un halogène et les mélanges des solvants mentionnés ci-dessus, et dans lequel
• le solvant éventuellement présent dans le liquide de refroidissement utilisé dans l'étape (iv), du moment qu'il est présent, et
• le solvant contenu dans le courant de solvant liquide utilisé comme liquide d'extraction par lavage dans l'étape (viii-2), du moment qu'elle soit mise en œuvre,
sont chacun les mêmes que le solvant contenu dans le courant de solvant liquide envoyé dans l'étape (v) dans la zone d'accumulation ou dans le réservoir.

14. Procédé selon l'une des revendications précédentes, dans lequel l'amine primaire est choisie dans le groupe consistant en la toluylènediamine, la diphénylméthanediamine, la xylylènediamine, la 1,5-pentanediamine, la 1,6-hexaméthylènediamine, l'isophoronediamine, le diaminodicyclohexylméthane et les mélanges des composés mentionnés ci-dessus.

15. Procédé selon l'une des revendications précédentes, dans lequel, dans toutes les étapes du procédé dans lesquelles on utilise un solvant organique, on utilise le même solvant organique, et ce solvant organique est choisi dans le groupe consistant en les hydrocarbures aliphatiques, les hydrocarbures aliphatiques sans substitution par un halogène, les hydrocarbures aromatiques avec substitution par un halogène et les mélanges des solvants organiques mentionnés ci-dessus.
